# EUROPEAN PATENT APPLICATION

(11) **EP 2 078 728 A1**
(43) Date of publication of application: **15.07.2009**
(21) Application number: 08100421.0
(22) Date of filing: 14.01.2008
(51) Int. Cl.: C07K 14/47, G01N 33/574, G01N 33/68

(54) **Novel isoform of versican and use in diagnosis and therapy**

(71) Applicant: Université de Liège, 4031 Angleur (BE)
(72) Inventor: Castronovo, Vincent, Prof. Université de Liège, 4000 Liège (BE); Kischel, Philippe, Dr. Université de Liège, 4000 Liège (BE)

(57) **Abstract**

An isolated and purified nucleic acid molecule having a nucleic acid sequence encoding the V4 isoform of versican, or a fragment thereof of at least 30 nucleotides comprising the exon boundary between exons 8a and 9 and a method and a kit for determining the presence and/or status of a tumor in a tissue of a patient by determining the presence and/or level of gene transcription of the V4 isoform of versican in such tissue. The V4 isoform of versican or a fragment thereof can also be used as a medicament, particularly in the treatment of breast cancer.

SEQUENCE LISTING

<110> Université de Liège

<120> Novel Isoforms of Versican and use in Diagnosis and Therapy

<130> 2007-17EP

<160> 30

<170> PatentIn version 3.3

<210> 1
<211> 3162
<212> DNA
<213> Homo sapiens


<220>
<221> CDS
<222> (1)..(3162)

<400> 1
atg ttc ata aat ata aag agc atc tta tgg atg tgt tca acc tta ata 48
Met Phe Ile Asn Ile Lys Ser Ile Leu Trp Met Cys Ser Thr Leu Ile
1               5                   10                  15

gta acc cat gcg cta cat aaa gtc aaa gtg gga aaa agc cca ccg gtg 96
Val Thr His Ala Leu His Lys Val Lys Val Gly Lys Ser Pro Pro Val
            20                  25                  30

agg ggc tcc ctc tct gga aaa gtc agc cta cct tgt cat ttt tca acg 144
Arg Gly Ser Leu Ser Gly Lys Val Ser Leu Pro Cys His Phe Ser Thr
        35                  40                  45

atg cct act ttg cca ccc agt tac aac acc agt gaa ttt ctc cgc atc 192
Met Pro Thr Leu Pro Pro Ser Tyr Asn Thr Ser Glu Phe Leu Arg Ile
    50                  55                  60

aaa tgg tct aag att gaa gtg gac aaa aat gga aaa gat ttg aaa gag 240
Lys Trp Ser Lys Ile Glu Val Asp Lys Asn Gly Lys Asp Leu Lys Glu
65                  70                  75                  80

act act gtc ctt gtg gcc caa aat gga aat atc aag att ggt cag gac 288
Thr Thr Val Leu Val Ala Gln Asn Gly Asn Ile Lys Ile Gly Gln Asp
                85                  90                  95

tac aaa ggg aga gtg tct gtg ccc aca cat ccc gag gct gtg ggc gat 336
Tyr Lys Gly Arg Val Ser Val Pro Thr His Pro Glu Ala Val Gly Asp
            100                 105                 110

gcc tcc ctc act gtg gtc aag ctg ctg gca agt gat gcg ggt ctt tac 384
Ala Ser Leu Thr Val Val Lys Leu Leu Ala Ser Asp Ala Gly Leu Tyr
        115                 120                 125

cgc tgt gac gtc atg tac ggg att gaa gac aca caa gac acg gtg tca 432
Arg Cys Asp Val Met Tyr Gly Ile Glu Asp Thr Gln Asp Thr Val Ser
    130                 135                 140

ctg act gtg gat ggg gtt gtg ttt cac tac agg gcg gca acc agc agg 480
Leu Thr Val Asp Gly Val Val Phe His Tyr Arg Ala Ala Thr Ser Arg
145                 150                 155                 160

tac aca ctg aat ttt gag gct gct cag aag gct tgt ttg gac gtt ggg 528
Tyr Thr Leu Asn Phe Glu Ala Ala Gln Lys Ala Cys Leu Asp Val Gly
                165                 170                 175

gca gtc ata gca act cca gag cag ctc ttt gct gcc tat gaa gat gga 576

Ala Val Ile Ala Thr Pro Glu Gln Leu Phe Ala Ala Tyr Glu Asp Gly
            180                 185                 190

ttt gag cag tgt gac gca ggc tgg ctg gct gat cag act gtc aga tat 624
Phe Glu Gln Cys Asp Ala Gly Trp Leu Ala Asp Gln Thr Val Arg Tyr
        195                 200                 205

ccc atc cgg gct ccc aga gta ggc tgt tat gga gat aag atg gga aag 672
Pro Ile Arg Ala Pro Arg Val Gly Cys Tyr Gly Asp Lys Met Gly Lys
    210                 215                 220

gca gga gtc agg act tat gga ttc cgt tct ccc cag gaa act tac gat 720
Ala Gly Val Arg Thr Tyr Gly Phe Arg Ser Pro Gln Glu Thr Tyr Asp
225                 230                 235                 240

gtg tat tgt tat gtg gat cat ctg gat ggt gat gtg ttc cac ctc act 768
Val Tyr Cys Tyr Val Asp His Leu Asp Gly Asp Val Phe His Leu Thr
                245                 250                 255

gtc ccc agt aaa ttc acc ttc gag gag gct gca aaa gag tgt gaa aac 816
Val Pro Ser Lys Phe Thr Phe Glu Glu Ala Ala Lys Glu Cys Glu Asn
            260                 265                 270

cag gat gcc agg ctg gca aca gtg ggg gaa ctc cag gcg gca tgg agg 864
Gln Asp Ala Arg Leu Ala Thr Val Gly Glu Leu Gln Ala Ala Trp Arg
        275                 280                 285

aac ggc ttt gac cag tgc gat tac ggg tgg ctg tcg gat gcc agc gtg 912
Asn Gly Phe Asp Gln Cys Asp Tyr Gly Trp Leu Ser Asp Ala Ser Val
    290                 295                 300

cgc cac cct gtg act gtg gcc agg gcc cag tgt gga ggt ggt cta ctt 960
Arg His Pro Val Thr Val Ala Arg Ala Gln Cys Gly Gly Gly Leu Leu
305                 310                 315                 320

ggg gtg aga acc ctg tat cgt ttt gag aac cag aca ggc ttc cct ccc 1008
Gly Val Arg Thr Leu Tyr Arg Phe Glu Asn Gln Thr Gly Phe Pro Pro
                325                 330                 335

cct gat agc aga ttt gat gcc tac tgc ttt aaa cgt cga atg agt gat 1056
Pro Asp Ser Arg Phe Asp Ala Tyr Cys Phe Lys Arg Arg Met Ser Asp
            340                 345                 350

ttg agt gta att ggt cat cca ata gat tca gaa tct aaa gaa gat gaa 1104
Leu Ser Val Ile Gly His Pro Ile Asp Ser Glu Ser Lys Glu Asp Glu
        355                 360                 365

cct tgt agt gaa gaa aca gat cca gtg cat gat cta atg gct gaa att 1152
Pro Cys Ser Glu Glu Thr Asp Pro Val His Asp Leu Met Ala Glu Ile
    370                 375                 380

tta cct gaa ttc cct gac ata att gaa ata gac cta tac cac agt gaa 1200
Leu Pro Glu Phe Pro Asp Ile Ile Glu Ile Asp Leu Tyr His Ser Glu
385                 390                 395                 400

gaa aat gaa gaa gaa gaa gaa gag tgt gca aat gct act gat gtg aca 1248
Glu Asn Glu Glu Glu Glu Glu Glu Cys Ala Asn Ala Thr Asp Val Thr
                405                 410                 415

acc acc cca tct gtg cag tac ata aat ggg aag cat ctc gtt acc act 1296
Thr Thr Pro Ser Val Gln Tyr Ile Asn Gly Lys His Leu Val Thr Thr
            420                 425                 430

gtg ccc aag gac cca gaa gct gca gaa gct agg cgt ggc cag ttt gaa 1344
Val Pro Lys Asp Pro Glu Ala Ala Glu Ala Arg Arg Gly Gln Phe Glu
        435                 440                 445

agt gtt gca cct tct cag aat ttc tcg gac agc tct gaa agt gat act 1392

Ser Val Ala Pro Ser Gln Asn Phe Ser Asp Ser Ser Glu Ser Asp Thr
    450                 455                 460

cat cca ttt gta ata gcc aaa acg gaa ttg tct act gct gtg caa cct 1440
His Pro Phe Val Ile Ala Lys Thr Glu Leu Ser Thr Ala Val Gln Pro
465                 470                 475                 480

aat gaa tct aca gaa aca act gag tct ctt gaa gtt aca tgg aag cct 1488
Asn Glu Ser Thr Glu Thr Thr Glu Ser Leu Glu Val Thr Trp Lys Pro
                485                 490                 495

gag act tac cct gaa aca tca gaa cat ttt tca ggt ggt gag cct gat 1536
Glu Thr Tyr Pro Glu Thr Ser Glu His Phe Ser Gly Gly Glu Pro Asp
            500                 505                 510

gtt ttc ccc aca gtc cca ttc cat gag gaa ttt gaa agt gga aca gcc 1584
Val Phe Pro Thr Val Pro Phe His Glu Glu Phe Glu Ser Gly Thr Ala
        515                 520                 525

aaa aaa ggg gca gaa tca gtc aca gag aga gat act gaa gtt ggt cat 1632
Lys Lys Gly Ala Glu Ser Val Thr Glu Arg Asp Thr Glu Val Gly His
    530                 535                 540

cag gca cat gaa cat act gaa cct gta tct ctg ttt cct gaa gag tct 1680
Gln Ala His Glu His Thr Glu Pro Val Ser Leu Phe Pro Glu Glu Ser
545                 550                 555                 560

tca gga gag att gcc att gac caa gaa tct cag aaa ata gcc ttt gca 1728
Ser Gly Glu Ile Ala Ile Asp Gln Glu Ser Gln Lys Ile Ala Phe Ala
                565                 570                 575

agg gct aca gaa gta aca ttt ggt gaa gag gta gaa aaa agt act tct 1776
Arg Ala Thr Glu Val Thr Phe Gly Glu Glu Val Glu Lys Ser Thr Ser
            580                 585                 590

gtc aca tac act ccc act ata gtt cca agt tct gca tca gca tat gtt 1824
Val Thr Tyr Thr Pro Thr Ile Val Pro Ser Ser Ala Ser Ala Tyr Val
        595                 600                 605

tca gag gaa gaa gca gtt acc cta ata gga aat cct tgg cca gat gac 1872
Ser Glu Glu Glu Ala Val Thr Leu Ile Gly Asn Pro Trp Pro Asp Asp
    610                 615                 620

ctg ttg tct acc aaa gaa agc tgg gta gaa gca act cct aga caa gtt 1920
Leu Leu Ser Thr Lys Glu Ser Trp Val Glu Ala Thr Pro Arg Gln Val
625                 630                 635                 640

gta gag ctc tca ggg agt tct tcg att cca att aca gaa ggc tct gga 1968
Val Glu Leu Ser Gly Ser Ser Ser Ile Pro Ile Thr Glu Gly Ser Gly
                645                 650                 655

gaa gca gaa gaa gat gaa gat aca atg ttc acc atg gta act gat tta 2016
Glu Ala Glu Glu Asp Glu Asp Thr Met Phe Thr Met Val Thr Asp Leu
            660                 665                 670

tca cag aga aat act act gat aca ctc att act tta gac act agc agg 2064
Ser Gln Arg Asn Thr Thr Asp Thr Leu Ile Thr Leu Asp Thr Ser Arg
        675                 680                 685

ata atc aca gaa agc ttt ttt gag gtt cct gca acc acc att tat cca 2112
Ile Ile Thr Glu Ser Phe Phe Glu Val Pro Ala Thr Thr Ile Tyr Pro
    690                 695                 700

gtt tct gaa caa cct tct gca aaa gtg gtg cct acc aag ttt gta agt 2160
Val Ser Glu Gln Pro Ser Ala Lys Val Val Pro Thr Lys Phe Val Ser
705                 710                 715                 720

gaa aca gac act tct gag tgg att tcc agt acc act gtt gag gaa aag 2208

Glu Thr Asp Thr Ser Glu Trp Ile Ser Ser Thr Thr Val Glu Glu Lys
                725                 730                 735

aaa agg aag gag gag gag gga act aca gga cct gat cgc tgc aaa atg      2256
Lys Arg Lys Glu Glu Glu Gly Thr Thr Gly Pro Asp Arg Cys Lys Met
            740                 745                 750

aac ccg tgc ctt aac gga ggc acc tgt tat cct act gaa act tcc tac      2304
Asn Pro Cys Leu Asn Gly Gly Thr Cys Tyr Pro Thr Glu Thr Ser Tyr
        755                 760                 765

gta tgc acc tgt gtg cca gga tac agc gga gac cag tgt gaa ctt gat      2352
Val Cys Thr Cys Val Pro Gly Tyr Ser Gly Asp Gln Cys Glu Leu Asp
    770                 775                 780

ttt gat gaa tgt cac tct aat ccc tgt cgt aat gga gcc act tgt gtt      2400
Phe Asp Glu Cys His Ser Asn Pro Cys Arg Asn Gly Ala Thr Cys Val
785                 790                 795                 800

gat ggt ttt aac aca ttc agg tgc ctc tgc ctt cca agt tat gtt ggt      2448
Asp Gly Phe Asn Thr Phe Arg Cys Leu Cys Leu Pro Ser Tyr Val Gly
                805                 810                 815

gca ctt tgt gag caa gat acc gag aca tgt gac tat ggc tgg cac aaa      2496
Ala Leu Cys Glu Gln Asp Thr Glu Thr Cys Asp Tyr Gly Trp His Lys
            820                 825                 830

ttc caa ggg cag tgc tac aaa tac ttt gcc cat cga cgc aca tgg gat      2544
Phe Gln Gly Gln Cys Tyr Lys Tyr Phe Ala His Arg Arg Thr Trp Asp
        835                 840                 845

gca gct gaa cgg gaa tgc cgt ctg cag ggt gcc cat ctc aca agc atc      2592
Ala Ala Glu Arg Glu Cys Arg Leu Gln Gly Ala His Leu Thr Ser Ile
    850                 855                 860

ctg tct cac gaa gaa caa atg ttt gtt aat cgt gtg ggc cat gat tat      2640
Leu Ser His Glu Glu Gln Met Phe Val Asn Arg Val Gly His Asp Tyr
865                 870                 875                 880

cag tgg ata ggc ctc aat gac aag atg ttt gag cat gac ttc cgt tgg      2688
Gln Trp Ile Gly Leu Asn Asp Lys Met Phe Glu His Asp Phe Arg Trp
                885                 890                 895

act gat ggc agc aca ctg caa tac gag aat tgg aga ccc aac cag cca      2736
Thr Asp Gly Ser Thr Leu Gln Tyr Glu Asn Trp Arg Pro Asn Gln Pro
            900                 905                 910

gac agc ttc ttt tct gct gga gaa gac tgt gtt gta atc att tgg cat      2784
Asp Ser Phe Phe Ser Ala Gly Glu Asp Cys Val Val Ile Ile Trp His
        915                 920                 925

gag aat ggc cag tgg aat gat gtt ccc tgc aat tac cat ctc acc tat      2832
Glu Asn Gly Gln Trp Asn Asp Val Pro Cys Asn Tyr His Leu Thr Tyr
    930                 935                 940

acg tgc aag aaa gga aca gtc gct tgc ggc cag ccc cct gtt gta gaa      2880
Thr Cys Lys Lys Gly Thr Val Ala Cys Gly Gln Pro Pro Val Val Glu
945                 950                 955                 960

aat gcc aag acc ttt gga aag atg aaa cct cgt tat gaa atc aac tcc      2928
Asn Ala Lys Thr Phe Gly Lys Met Lys Pro Arg Tyr Glu Ile Asn Ser
                965                 970                 975

ctg att aga tac cac tgc aaa gat ggt ttc att caa cgt cac ctt cca      2976
Leu Ile Arg Tyr His Cys Lys Asp Gly Phe Ile Gln Arg His Leu Pro
            980                 985                 990

act atc cgg tgc tta gga aat gga aga tgg gct ata cct aaa att acc      3024

Thr Ile Arg Cys Leu Gly Asn Gly Arg Trp Ala Ile Pro Lys Ile Thr
        995                 1000                1005

tgc atg aac cca tct gca tac caa agg act tat tct atg aaa tac 3069
Cys Met Asn Pro Ser Ala Tyr Gln Arg Thr Tyr Ser Met Lys Tyr
    1010                1015                1020

ttt aaa aat tcc tca tca gca aag gac aat tca ata aat aca tcc 3114
Phe Lys Asn Ser Ser Ser Ala Lys Asp Asn Ser Ile Asn Thr Ser
    1025                1030                1035

aaa cat gat cat cgt tgg agc cgg agg tgg cag gag tcg agg cgc 3159
Lys His Asp His Arg Trp Ser Arg Arg Trp Gln Glu Ser Arg Arg
    1040                1045                1050

tga 3162

<210> 2
<211> 1053
<212> PRT
<213> Homo sapiens

<400> 2

Met Phe Ile Asn Ile Lys Ser Ile Leu Trp Met Cys Ser Thr Leu Ile
1               5                   10                  15

Val Thr His Ala Leu His Lys Val Lys Val Gly Lys Ser Pro Pro Val
            20                  25                  30

Arg Gly Ser Leu Ser Gly Lys Val Ser Leu Pro Cys His Phe Ser Thr
        35                  40                  45

Met Pro Thr Leu Pro Pro Ser Tyr Asn Thr Ser Glu Phe Leu Arg Ile
    50                  55                  60

Lys Trp Ser Lys Ile Glu Val Asp Lys Asn Gly Lys Asp Leu Lys Glu
65                  70                  75                  80

Thr Thr Val Leu Val Ala Gln Asn Gly Asn Ile Lys Ile Gly Gln Asp
                85                  90                  95

Tyr Lys Gly Arg Val Ser Val Pro Thr His Pro Glu Ala Val Gly Asp
            100                 105                 110

Ala Ser Leu Thr Val Val Lys Leu Leu Ala Ser Asp Ala Gly Leu Tyr
        115                 120                 125

Arg Cys Asp Val Met Tyr Gly Ile Glu Asp Thr Gln Asp Thr Val Ser
    130                 135                 140

Leu Thr Val Asp Gly Val Val Phe His Tyr Arg Ala Ala Thr Ser Arg
145                 150                 155                 160

Tyr Thr Leu Asn Phe Glu Ala Ala Gln Lys Ala Cys Leu Asp Val Gly
                165                 170                 175

Ala Val Ile Ala Thr Pro Glu Gln Leu Phe Ala Ala Tyr Glu Asp Gly
            180                 185                 190

Phe Glu Gln Cys Asp Ala Gly Trp Leu Ala Asp Gln Thr Val Arg Tyr
        195                 200                 205

Pro Ile Arg Ala Pro Arg Val Gly Cys Tyr Gly Asp Lys Met Gly Lys
    210                 215                 220

Ala Gly Val Arg Thr Tyr Gly Phe Arg Ser Pro Gln Glu Thr Tyr Asp
225                 230                 235                 240

Val Tyr Cys Tyr Val Asp His Leu Asp Gly Asp Val Phe His Leu Thr
                245                 250                 255

Val Pro Ser Lys Phe Thr Phe Glu Glu Ala Ala Lys Glu Cys Glu Asn
            260                 265                 270

Gln Asp Ala Arg Leu Ala Thr Val Gly Glu Leu Gln Ala Ala Trp Arg
        275                 280                 285

Asn Gly Phe Asp Gln Cys Asp Tyr Gly Trp Leu Ser Asp Ala Ser Val
    290                 295                 300

Arg His Pro Val Thr Val Ala Arg Ala Gln Cys Gly Gly Gly Leu Leu
305                 310                 315                 320

Gly Val Arg Thr Leu Tyr Arg Phe Glu Asn Gln Thr Gly Phe Pro Pro
                325                 330                 335

Pro Asp Ser Arg Phe Asp Ala Tyr Cys Phe Lys Arg Arg Met Ser Asp
            340                 345                 350

Leu Ser Val Ile Gly His Pro Ile Asp Ser Glu Ser Lys Glu Asp Glu
        355                 360                 365

Pro Cys Ser Glu Glu Thr Asp Pro Val His Asp Leu Met Ala Glu Ile
    370                 375                 380

Leu Pro Glu Phe Pro Asp Ile Ile Glu Ile Asp Leu Tyr His Ser Glu
385                 390                 395                 400

Glu Asn Glu Glu Glu Glu Glu Glu Cys Ala Asn Ala Thr Asp Val Thr
                405                 410                 415

Thr Thr Pro Ser Val Gln Tyr Ile Asn Gly Lys His Leu Val Thr Thr
            420                 425                 430

Val Pro Lys Asp Pro Glu Ala Ala Glu Ala Arg Arg Gly Gln Phe Glu
        435                 440                 445

Ser Val Ala Pro Ser Gln Asn Phe Ser Asp Ser Ser Glu Ser Asp Thr
    450                 455                 460

His Pro Phe Val Ile Ala Lys Thr Glu Leu Ser Thr Ala Val Gln Pro
465                 470                 475                 480

Asn Glu Ser Thr Glu Thr Thr Glu Ser Leu Glu Val Thr Trp Lys Pro
                485                 490                 495

Glu Thr Tyr Pro Glu Thr Ser Glu His Phe Ser Gly Gly Glu Pro Asp
            500                 505                 510

Val Phe Pro Thr Val Pro Phe His Glu Glu Phe Glu Ser Gly Thr Ala
        515                 520                 525

Lys Lys Gly Ala Glu Ser Val Thr Glu Arg Asp Thr Glu Val Gly His
    530                 535                 540

Gln Ala His Glu His Thr Glu Pro Val Ser Leu Phe Pro Glu Glu Ser
545                 550                 555                 560

Ser Gly Glu Ile Ala Ile Asp Gln Glu Ser Gln Lys Ile Ala Phe Ala
                565                 570                 575

Arg Ala Thr Glu Val Thr Phe Gly Glu Glu Val Glu Lys Ser Thr Ser
            580                 585                 590

Val Thr Tyr Thr Pro Thr Ile Val Pro Ser Ser Ala Ser Ala Tyr Val
        595                 600                 605

Ser Glu Glu Glu Ala Val Thr Leu Ile Gly Asn Pro Trp Pro Asp Asp
    610                 615                 620

Leu Leu Ser Thr Lys Glu Ser Trp Val Glu Ala Thr Pro Arg Gln Val
625                 630                 635                 640

Val Glu Leu Ser Gly Ser Ser Ser Ile Pro Ile Thr Glu Gly Ser Gly
                645                 650                 655

Glu Ala Glu Glu Asp Glu Asp Thr Met Phe Thr Met Val Thr Asp Leu
            660                 665                 670

Ser Gln Arg Asn Thr Thr Asp Thr Leu Ile Thr Leu Asp Thr Ser Arg
        675                 680                 685

Ile Ile Thr Glu Ser Phe Phe Glu Val Pro Ala Thr Thr Ile Tyr Pro
    690                 695                 700

Val Ser Glu Gln Pro Ser Ala Lys Val Val Pro Thr Lys Phe Val Ser
705                 710                 715                 720

Glu Thr Asp Thr Ser Glu Trp Ile Ser Ser Thr Thr Val Glu Glu Lys
                725                 730                 735

Lys Arg Lys Glu Glu Glu Gly Thr Thr Gly Pro Asp Arg Cys Lys Met
            740                 745                 750

Asn Pro Cys Leu Asn Gly Gly Thr Cys Tyr Pro Thr Glu Thr Ser Tyr
        755                 760                 765

Val Cys Thr Cys Val Pro Gly Tyr Ser Gly Asp Gln Cys Glu Leu Asp
    770                 775                 780

Phe Asp Glu Cys His Ser Asn Pro Cys Arg Asn Gly Ala Thr Cys Val
785                 790                 795                 800

Asp Gly Phe Asn Thr Phe Arg Cys Leu Cys Leu Pro Ser Tyr Val Gly
                805                 810                 815

Ala Leu Cys Glu Gln Asp Thr Glu Thr Cys Asp Tyr Gly Trp His Lys
            820                 825                 830

Phe Gln Gly Gln Cys Tyr Lys Tyr Phe Ala His Arg Arg Thr Trp Asp
        835                 840                 845

Ala Ala Glu Arg Glu Cys Arg Leu Gln Gly Ala His Leu Thr Ser Ile
    850                 855                 860

Leu Ser His Glu Glu Gln Met Phe Val Asn Arg Val Gly His Asp Tyr
865                 870                 875                 880

Gln Trp Ile Gly Leu Asn Asp Lys Met Phe Glu His Asp Phe Arg Trp
                885                 890                 895

Thr Asp Gly Ser Thr Leu Gln Tyr Glu Asn Trp Arg Pro Asn Gln Pro
            900                 905                 910

Asp Ser Phe Phe Ser Ala Gly Glu Asp Cys Val Val Ile Ile Trp His
        915                 920                 925

Glu Asn Gly Gln Trp Asn Asp Val Pro Cys Asn Tyr His Leu Thr Tyr
    930                 935                 940

Thr Cys Lys Lys Gly Thr Val Ala Cys Gly Gln Pro Pro Val Val Glu
945                 950                 955                 960

Asn Ala Lys Thr Phe Gly Lys Met Lys Pro Arg Tyr Glu Ile Asn Ser
                965                 970                 975

Leu Ile Arg Tyr His Cys Lys Asp Gly Phe Ile Gln Arg His Leu Pro
            980                 985                 990

Thr Ile Arg Cys Leu Gly Asn Gly Arg Trp Ala Ile Pro Lys Ile Thr
        995                 1000                1005

Cys Met Asn Pro Ser Ala Tyr Gln Arg Thr Tyr Ser Met Lys Tyr
    1010                1015                1020

Phe Lys Asn Ser Ser Ser Ala Lys Asp Asn Ser Ile Asn Thr Ser
    1025                1030                1035

Lys His Asp His Arg Trp Ser Arg Arg Trp Gln Glu Ser Arg Arg
    1040                1045                1050

<210> 3
<211> 18
<212> DNA
<213> homo sapiens

<400> 3
gaactacagg tacggctt 18

<210> 4
<211> 1194
<212> DNA
<213> homo sapiens

<400> 4
gtcgaatgag tgatttgagt gtaattggtc atccaataga ttcagaatct aaagaagatg 60

aaccttgtag tgaagaaaca gatccagtgc atgatctaat ggctgaaatt ttacctgaat 120

tccctgacat aattgaaata gacctatacc acagtgaaga aaatgaagaa gaagaagaag 180

agtgtgcaaa tgctactgat gtgacaacca ccccatctgt gcagtacata aatgggaagc 240

atctcgttac cactgtgccc aaggacccag aagctgcaga agctaggcgt ggccagtttg 300

aaagtgttgc accttctcag aatttctcgg acagctctga aagtgatact catccatttg 360

taatagccaa aacggaattg tctactgctg tgcaacctaa tgaatctaca gaaacaactg 420

agtctcttga agttacatgg aagcctgaga cttaccctga aacatcagaa catttttcag 480

gtggtgagcc tgatgttttc cccacagtcc cattccatga ggaatttgaa agtggaacag 540

ccaaaaaagg ggcagaatca gtcacagaga gagatactga agttggtcat caggcacatg 600

aacatactga acctgtatct ctgtttcctg aagagtcttc aggagagatt gccattgacc 660

aagaatctca gaaaatagcc tttgcaaggg ctacagaagt aacatttggt gaagaggtag 720

aaaaaagtac ttctgtcaca tacactccca ctatagttcc aagttctgca tcagcatatg 780

tttcagagga agaagcagtt accctaatag gaaatccttg gccagatgac ctgttgtcta 840

ccaaagaaag ctgggtagaa gcaactccta gacaagttgt agagctctca gggagttctt 900

cgattccaat tacagaaggc tctggagaag cagaagaaga tgaagataca atgttcacca 960

tggtaactga tttatcacag agaaatacta ctgatacact cattacttta gacactagca 1020

ggataatcac agaaagcttt tttgaggttc ctgcaaccac catttatcca gtttctgaac 1080

aaccttctgc aaaagtggtg cctaccaagt ttgtaagtga aacagacact tctgagtgga 1140

tttccagtac cactgttgag gaaaagaaaa ggaaggagga ggagggaact acag 1194

<210> 5
<211> 30
<212> DNA
<213> artificial

<220>
<223> primer

<400> 5
caccatgttc ataaatataa agagcatctt 30

<210> 6
<211> 30
<212> DNA
<213> Artificial

<220>
<223> primer

<400> 6
tcagcgcctc gactcctgcc acctccggct 30

<210> 7
<211> 30
<212> DNA
<213> Artificial

<220>
<223> primer

<400> 7
gcgcctcgac tcctgccacc tccggctcca 30

<210> 8
<211> 19
<212> DNA
<213> artificial

<220>
<223> primer

<400> 8
aggcacgggt tcattttgc 19

<210> 9
<211> 20
<212> DNA
<213> artificial

<220>
<223> primer

<400> 9
ccctccccct gatagcagat 20

<210> 10
<211> 27
<212> DNA

<213>  artificial

<220>
<223>  primer

<400>  10
ttagattctg aatctattgg atgacca                                          27


<210>  11
<211>  26
<212>  DNA
<213>  artificial

<220>
<223>  primer

<400>  11
cagtaccact gttgaggaaa agaaaa                                           26


<210>  12
<211>  31
<212>  DNA
<213>  artificial

<220>
<223>  primer

<400>  12
tcattttgca gcgatcaggt cctgtagttc c                                     31


<210>  13
<211>  22
<212>  DNA
<213>  artificial

<220>
<223>  primer

<400>  13
gcacaaaatt tcaccctgac at                                               22


<210>  14
<211>  35
<212>  DNA
<213>  artificial

<220>
<223>  primer

<400>  14
actcaaatca ctcattcgac ctgtcttatt ttctc                                 35


<210>  15
<211>  27
<212>  DNA
<213>  artificial

<220>
<223>  primer

<400>  15
ttagattctg aatctattgg atgacca                                          27

<210>  16
<211>  19
<212>  DNA
<213>  artificial

<220>
<223>  primer

<400>  16
ggcttccctc cccctgata                                                    19


<210>  17
<211>  33
<212>  DNA
<213>  artificial

<220>
<223>  probe

<400>  17
ttgatgccta ctgctttaaa cgtcgaatga gtg                                    33


<210>  18
<211>  30
<212>  DNA
<213>  artificial

<220>
<223>  primer

<400>  18
cttctttaga ttctgaatct attggatgac                                        30


<210>  19
<211>  25
<212>  DNA
<213>  artificial

<220>
<223>  primer

<400>  19
gcattaatga agagtcagtg gaagg                                             25


<210>  20
<211>  29
<212>  DNA
<213>  artificial

<220>
<223>  probe

<400>  20
cggcaatcta tttaccagga cctgatcgc                                         29


<210>  21
<211>  21
<212>  DNA
<213>  artificial

<220>
<223>  primer

<400> 21
ttaaggcacg ggttcatttt g 21

<210> 22
<211> 22
<212> DNA
<213> artificial

<220>
<223> primer

<400> 22
cccagcaagc acaaaatttc ac 22

<210> 23
<211> 27
<212> DNA
<213> artificial

<220>
<223> probe

<400> 23
ttcattttgc agcgatcagg tcctgtc 27

<210> 24
<211> 22
<212> DNA
<213> artificial

<220>
<223> primer

<400> 24
taggataaca ggtgcctccg tt 22

<210> 25
<211> 20
<212> DNA
<213> artificial

<220>
<223> primer

<400> 25
ccctccccct gatagcagat 20

<210> 26
<211> 28
<212> DNA
<213> artificial

<220>
<223> probe

<400> 26
tgcctactgc tttaaacgac ctgatcgc 28

<210> 27
<211> 18
<212> DNA

<213>  artificial

<220>
<223>  primer

<400>  27
   ggcacgggtt cattttgc                                                    18


<210>  28
<211>  26
<212>  DNA
<213>  artificial

<220>
<223>  primer

<400>  28
cagtaccact gttgaggaaa agaaaa                                            26


<210>  29
<211>  26
<212>  DNA
<213>  artificial

<220>
<223>  probe

<400>  29
tgcagcgatc aggtcctgta gttccc                                            26


<210>  30
<211>  20
<212>  DNA
<213>  artificial

<220>
<223>  primer

<400>  30
cgttaaggca cgggttcatt                                                   20

## Description

### FIELD OF THE INVENTION

The invention described herein relates to the identification and cloning of a novel isoform of versican and the use thereof in diagnosis and therapy.

### BACKGROUND

Versican, the large "versatile proteoglycan", is a member of the lectican/hyalectin family composed of large aggregating proteoglycans, including also aggrecan, neurocan and brevican. Versican is a large chondroitin sulfate proteoglycan that contributes to key events in development and diseases. Four main versican isoforms have been characterized, namely the three glycosaminoglycan (GAG) bearing isoforms V0, V1 and V2 and the shorter V3 isoform.

Versican is the major chondroitin sulfate proteoglycan (CSPG) of the vessel wall. It is characterized by a number of important structural features at the DNA and/or protein level. The genomic structure of the versican gene (also known as CSPG2 gene) is highly similar to other proteoglycan genes. CSPG2 encodes, within 15 exons (Accession No. NM_004385), the large (3396 amino acids) versican V0 protein (Naso et al., 1994). The structure of the versican protein consists of a glycosaminoglycan (GAG) attachment domain of variable size, surrounded by two external globular domains, G1 and G3. The central extended region with attached glycosaminoglycan (GAG) chains confers space filling and viscoelastic properties on the matrix. The chondroitin sulfate (CS) chains are highly negatively charged and are believed to contribute anti-adhesive properties to the molecule. The amino- and carboxy-terminal globular domains are domains through which versican binds to other matrix molecules.

The amino-terminal G1 domain is composed of Ig repeats and a hyaluronan binding domain. This domain is responsible for the binding of versican to the glycosaminoglycan hyaluronan (HA) (LeBaron et al., 1992). Together, these molecules can form large pericellular coats, which are anchored to cells via HA receptors, and which may inhibit the interaction of the cell with other cells or molecules.

The carboxyterminal selectin-like G3 domain is composed of EGF, lectin and complement regulatory protein elements (Margolis and Margolis, 1994). The EGF-like domain has been shown to be proproliferative and the lectin-like domain binds tenascin-R and fibulin- 1.

The versican gene has been shown to generate different splice variants. The alternate splicing of two exons, namely GAGα (encoded by exon 7) and GAGβ (encoded by exon 8), generates three variants, V1 (Zimmermann and Ruoslahti, 1989), V2 (Dours-Zimmermann and Zimmermann, 1994) and V3 (Zako et al., 1995). While the full length V0 isoform (Accession No. U16306) contains both GAGα and GAGβ exons, V1 (Accession No. X15998) and V2 (Accession No. U26555) isoforms lack GAGα and GAGβ, respectively. These isoforms thus differ in the length of the central domain and are predicted to also differ in the number of CS chains attached. A fourth variant, V3 (Accession No. D32039), lacks both GAG (chondroitin sulfate) attachment regions (Zako et al., 1995), resulting thereby in a side chain polymorphism in the different versican variants. As it comprises only the two globular domains, it is thus predicted to be a glycoprotein, but not a proteoglycan.

Versican is expressed by a series of cultured cells, including fibroblasts (e.g. human fetal lung fibroblasts, skin and gingiva fibroblasts, among others), proliferating keratinocytes, arterial smooth muscle cells, and endothelial cells. In normal tissues, versican is mainly detected in the aortic extracellular matrix, in nervous tissues, in the stratum basale of the epidermis, and in the placenta. While the expression of versican transcripts in human tissues has been investigated (Cattaruzza et al., 2002), information about protein expression levels of the different isoforms in different tissues has remained scarce, especially for the lower molecular weight V3 isoform. V0 and V1 are usually documented as the main isoforms found in tissues reported to express versican, while V2 is the main and restricted isoform in the mature brain.

### SUMMARY OF THE INVENTION

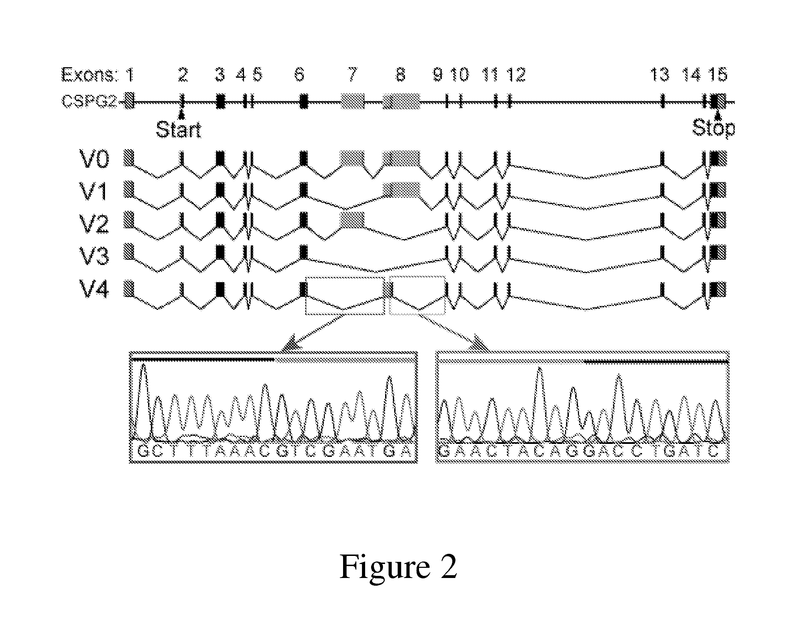

The present invention is based on the observation that expression of versican isoforms is significantly increased both at the protein and mRNA levels in human breast cancer tissues, as compared with non-tumoral adjacent tissues. A new versican variant has unexpectedly been found. Sequence analysis of this new variant revealed that the first 1194bp of exon 8 (GAGβ) are sandwiched between the end of exon 6 and the beginning of exon 9 (Fig. 2). This new variant, generated by alternate splicing involving the classical GT-AG rule for splicing junctions, has been termed "V4". The V4 isoform is of interest for the diagnosis and treatment of tumours, in particular breast cancer tumours.

One aspect of the invention provides isolated and purified nucleic acid molecules having a nucleic acid sequence encoding the V4 isoform of versican, or a fragment thereof comprising the exon boundary between exons 8a and 9. Particular embodiments relate to nucleic acid molecules of at least 30, more particularly of at least 100 nucleotides. In further particular embodiments, nucleic acid molecules of the invention have the sequence of SEQ ID NO:1 or a sequence having at least 70%, more particularly at least 80%, most particularly at least 95% sequence identity therewith.

A further aspect of the invention provides methods of determining the presence and/or status of a tumor in a tissue of a patient, comprising the step of determining in the tissue the presence and/or level of gene transcription of the V4 isoform of versican, whereby the presence and/or level of gene transcription of the V4 isoform of versican is indicative of the presence and/or status of the tumor. In particular embodiments, methods of this aspect of the invention comprise, comparing the level of gene transcription of the V4 isoform of versican in a sample of the tissue with the level of gene transcription in a control sample, wherein upregulation of gene transcription as compared to control is indicative of tumor formation. More particularly in such methods upregulation of gene transcription compared to control of at least 20% is indicative of tumor formation.

In further particular embodiments, methods are provided which further comprise, in addition to determining the level of transcription of the V4 isoform, determining the presence and/or level of gene transcription of the V0, V1, V2 and/or V3 isoform of versican, whereby the presence and/or level of gene transcription of the V4 isoform of versican and one or more other isoforms of versican are indicative of the presence and/or status of the tumor.

In particular embodiments of methods for determining the presence and/or status of a tumor in a tissue of a patient based on the presence and/or level of gene transcription of the V4 isoform of versican described herein, the presence and/or level of gene transcription of the V4 isoform of versican is determined either at the protein level or at the gene transcription level. In further particular embodiments of methods described herein, the presence and/or level of gene transcription is determined at the protein level using an anti-versican antibody or an antibody specific for the V4 isoform of versican, more particularly the anti-versican antibody 12c5 or an antigen-binding fragment thereof.

In further embodiments of methods for determining the presence and/or status of a tumor in a tissue of a patient based on the presence and/or level of gene transcription of the V4 isoform of versican the presence and/or level of gene transcription is determined at the gene transcription level. More particularly methods are envisaged wherein gene transcription is detected using at least one primer, probe or probe combination capable of specifically recognizing the V4 isoform of versican. Additionally or alternatively, the isoforms of versican are identified based on the size of PCR amplification products.

In particular embodiments of methods for determining the presence and/or status of a tumor in a tissue of a patient based on the presence and/or level of gene transcription of the V4 isoform of versican gene transcription is detected using a probe or a primer combination capable of specifically hybridizing to or amplifying the exon boundary between exons 8a and 9.

The above-described methods are of particular interest for determining the presence and/or status of a tumor in breast tissue.

In particular embodiments of methods for determining the presence and/or status of a tumor in a tissue of a patient based on the presence and/or level of gene transcription of the V4 isoform of versican the status of the tumor is further evaluated by determining the amount of glycosaminoglycan (GAG) attached to the V4 isoform of versican protein. In particular embodiments of methods described herein, the tissue envisaged is selected from the group consisting of blood, tissue biopsy material or autopsy material.

Yet a further aspect of the present invention provides methods for determining the presence and/or status of breast cancer in breast tissue of a patient, comprising the steps of specifically determining in a sample of the breast tissue the presence and/or level of gene transcription of at least one isoform of versican selected from the group consisting of the V0 isoform, the V1 isoform, the V2 isoform, the V3 isoform, and, the V4 isoform, and, based on the results, determining the presence and/or status of said tumor based on the result obtained therefrom. In particular embodiments of these methods, the presence and/or level of gene transcription is determined at the protein level.

In a further aspect of the invention, methods are provided for screening for an agent which modulates the expression of the V4 isoform of versican. In particular embodiments, such methods comprising contacting host cells which comprise a nucleic acid molecule encoding the V4 isoform of versican with candidate molecules; and determining whether the candidate molecules modulate the expression of the V4 isoform.

A further aspect of the invention provides oligonucleotides capable of specific recognition of the V4 isoform of versican by specifically hybridizing to the exon boundary between exons 8a and 9. Such oligonucleotides are of particular interest for use in the methods for determining V4 isoform expression described above. In addition, the invention provides kits for detecting the presence and/or status of a tumor in a tissue, comprising at least one of the following:
- a probe or primer pair capable of specifically detecting the V4 isoform of versican by specifically hybridizing to or amplifying the exon boundary between exons 8a and 9; or
- an antibody specific for the V4 isoform of versican.

A further aspect of the invention provides vectors comprising nucleic acid molecules having a nucleic acid sequence encoding the V4 isoform of versican, or a fragment thereof comprising the exon boundary between exons 8a and 9 described hereinabove. Particular embodiments relate to vectors comprising nucleic acid molecules of at least 100 nucleotides. In further particular embodiments, vectors are provided which comprise nucleic acid molecules having the sequence of SEQ ID NO:1 or a sequence having at least 95% sequence identity therewith. Such vectors are of particular use in the recombinant expression of the V4 isoform or fragments thereof.

Accordingly, yet a further aspect of the invention provides methods for producing the V4 isoform of versican or a fragment thereof of at least 100 nucleotides comprising the exon boundary between exons 8a and 9, comprising:
- providing a host cell;
- transforming the host cell with an isolated and purified nucleic acid molecule having a nucleic acid sequence encoding the V4 isoform of versican or a fragment thereof of at least 100 nucleotides comprising the exon boundary between exons 8a and 9;
- culturing the host cell so that the host cell expresses the V4 isoform of versican or the fragment thereof of at least 100 nucleotides comprising the exon boundary between exons 8a and 9.

Yet a further aspect of the invention relates to the therapeutic use of the V4 isoform of versican or a fragment thereof. Thus this aspect provides V4 isoform of versican or a fragment thereof for use as a medicament.

More particularly, the use of the V4 isoform of versican or a fragment thereof is envisaged in the treatment of cancer, most particularly breast cancer. Accordingly, the invention envisages the use of the V4 isoform of versican or a fragment thereof for the manufacture of a medicament for the treatment of breast cancer.

Yet another aspect of the invention relates to the therapeutic use of inhibitors of versican expression and/or function. Accordingly, the invention provides for inhibitors of the V4 isoform of versican for use as a medicament, wherein the inhibitor of the V4 isoform of versican is selected from an inhibitory antibody specific for the V4 isoform of versican, a small molecule inhibitor of the V4 isoform of versican or an antisense nucleic acid molecule directed against the V4 isoform of versican. Particular embodiments of the invention provide inhibitors of the V4 isoform of versican for use in the treatment of breast cancer, wherein the inhibitor of the V4 isoform of versican is selected from an inhibitory antibody specific for the V4 isoform of versican, a small molecule inhibitor of the V4 isoform of versican or an antisense nucleic acid molecule directed against the V4 isoform of versican. Accordingly, the invention envisages the use of an inhibitor of the V4 isoform of versican for the manufacture of a medicament for the treatment of breast cancer, wherein the inhibitor of the V4 isoform of versican is selected from an inhibitory antibody specific for the V4 isoform of versican, a small molecule inhibitor of the V4 isoform of versican or an antisense nucleic acid molecule directed against the V4 isoform of versican.

Yet a further aspect of the invention provides ligands binding specifically to the V4 isoform of versican. These are of particular interest in the therapy of diseases related to V4 isoform expression.

Yet a further aspect of the invention provides ligands binding specifically to the V4 isoform of versican which are coupled to a bioactive agent or diagnostic agent. Such ligands are of particular interest in the targeting of drugs or diagnostic agents.

Accordingly, the invention provides methods for targeting an anti-cancer drug to a breast tumor in a patient, which comprise the steps of linking the anti-cancer drug to a ligand specific for the V4 isoform of versican and administering the ligand so obtained to the patient.

The above and other characteristics, features and advantages of the present invention will become apparent from the following detailed description, taken in conjunction with the accompanying Figures, which illustrate, by way of example, the principles of the invention. This description is given for the sake of example only, without limiting the scope of the invention.

### BRIEF DESCRIPTION OF THE FIGURES

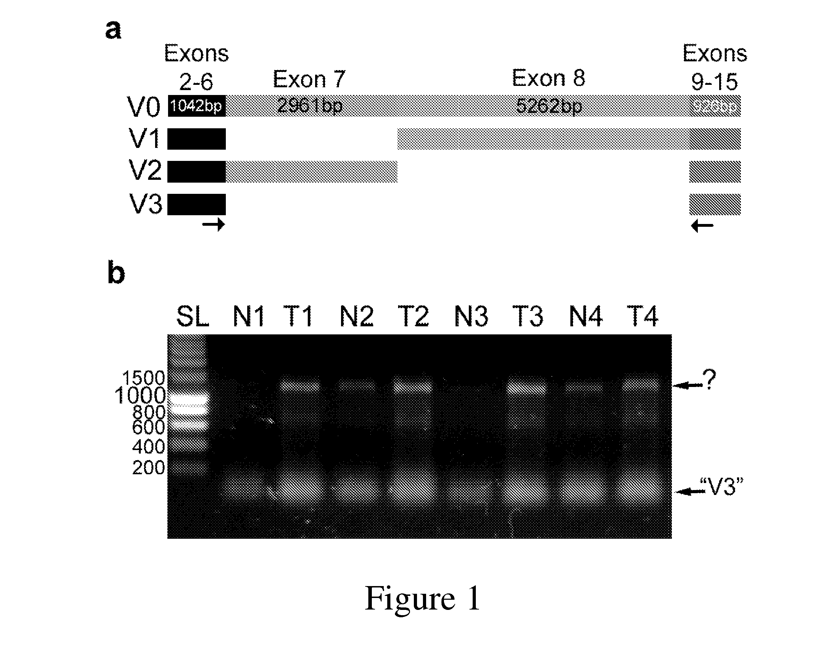

Figure 1: a: Schematic overview of the four different mRNA splice variants of versican known in the art (V0, V1, V2 and V3). The splice variants result from the alternate splicing of exons 7 and 8. The various splicing variants contain both (V0), one (V1 and V2) or neither of these two exons (V3). The size of the mRNA segments are indicated in each V0 exon (values from ATG start codon to the TGA stop codon). Arrows indicate the position of the forward and reverse primers used to generate a V3 amplicon with a size compatible with qRT-PCR.
   b: Representative RT-PCR detection of the V3 isoform transcript in normal breast tissues (Nx) and matched breast tumor lesions (Tx). Two different bands are detected in the tumor samples: the amplicon at the expected size (73bp), designated as "V3", and an additional band at ∼1200bp, designated as "?". This additional band is also faintly visible in N2 and N4. SL: SmartLadder (Eurogentec).
Figure 2: Genomic structure of the versican gene and scheme of the isoforms, including the new versican V4 isoform. The 15 exons of the versican gene are depicted as filled rectangles linked by introns depicted by black lines. 5'UTR (before ATG start codon) and 3'UTR (after TGA stop codon) are represented in dark grey. GAG exons (in light grey) represent GAGα (exon 7) and GAGβ (exon 8), respectively. The darker inset superimposed in the beginning of GAGβ represents the portion of exon 8 found in the V4 isoform. The left boxed electropherogram displays 18 nucleotides surrounding the junction between exon 6 and 8 (identical to the junction found in V1). The right boxed electropherogram displays 18 nucleotides surrounding the junction between the truncated exon 8 (referred to as exon 8a) and exon 9. The cryptic splice donor site inside exon 8 corresponds to the following underlined sequence: *GAACTAC**AGG*TACGGCTT - SEQ ID NO: 3).

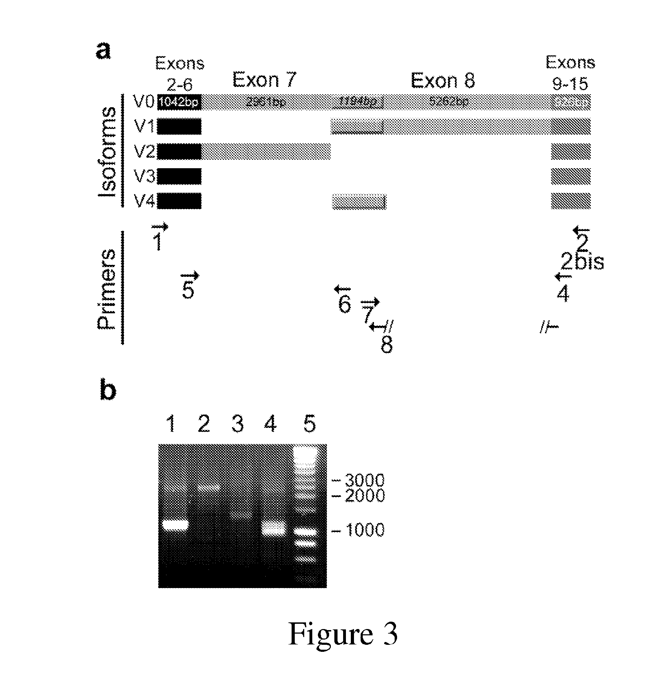

Figure 3: a: Schematic representation of the five versican isoforms, and relative position of the primers used for PCR detection and cloning of the V4 isoform in breast cancer tissues.
   b: RT-PCR amplification of different segments of the V4 isoform. PCR experiments using primers "1" and "2" produced only inconsistent V4 amplification (not shown), smaller fragments were thus amplified to ascertain the presence of a full length V4. Lane 1: PCR with primers "1" and "4" generated two fragments: one strong fragment at ∼1100bp (consistent with V3 amplification at 1077bp), and one ∼2300bp fragment (consistent with V4 amplification at 2271 bp). Lane 2: the use of primer "1" and the V4 specific primer "8" generated a ∼2300bp fragment corresponding to the V4 fragment (2261 bp). Lane 3: primers "5" and "4" primers generated a band at ∼1200bp corresponding to the V4 fragment (same as in Fig.1b and sequenced in Fig. 2). Lane 4: Primers "5" and "2" generated at least two specific products: the one at 1000bp is consistent with V3 amplification, and the band at ∼2200bp is consistent with the structure of V4 as depicted in Fig. 2 (expected size of 2160bp). Lane 5: SmartLadder (Eurogentec).

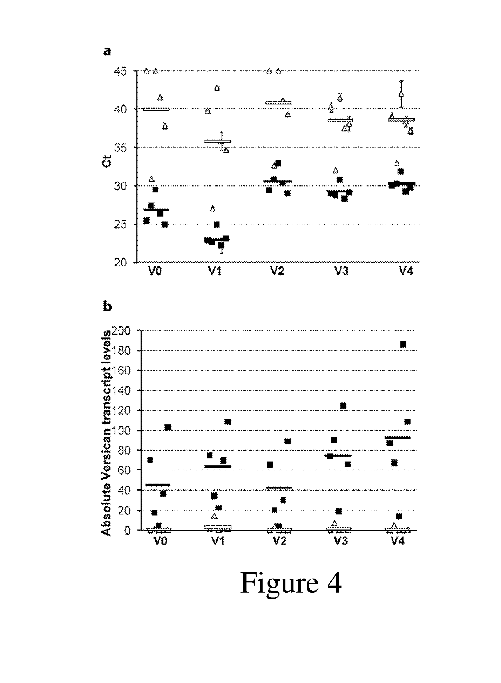

Figure 4: Quantitation of the different versican isoforms by quantitative real-time RT-PCR. Total RNA from malignant and matched adjacent normal breast tissues from five different patients was assayed in qRT-PCR with the primers and probes described in Table 1 b. Replicates were averaged, and values from normal and malignant breast tissues are represented by open triangles (Δ) and black filled squares (■), respectively. Averaged values from the five patients are represented by empty and black filled bars for normal and tumor samples, respectively.
   a: Ct values obtained for the five different versican isoforms, determined in normal breast and in adjacent tumor breast tissues.
   b: Expression of the absolute versican transcript levels (without normalisation using a house-keeping gene), as determined using a dilution curve.

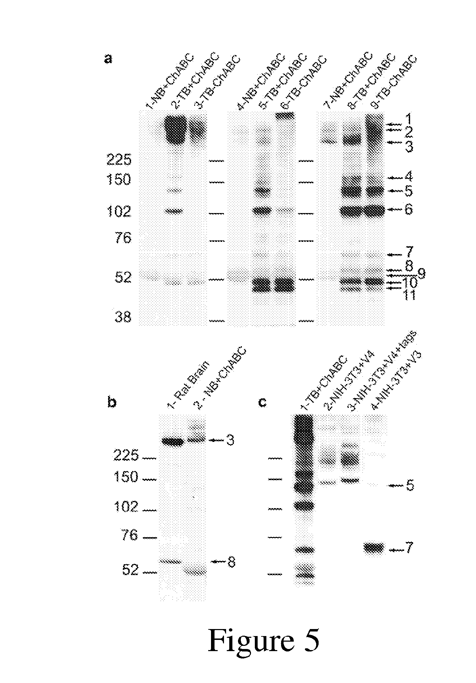

Figure 5: Western blots revealed with 12c5 anti-versican antibody.
   a: 4-12% PAGE of normal-tumor paired samples. Lanes [1, 2, 3], [4, 5, 6] and [7, 8, 9] correspond to three blocks containing samples in the respective order: [normal breast sample digested with chondroitinase ABC, associated tumor sample digested with chondroitinase ABC, associated tumor sample without chondroitinase ABC digestion]. See Example 3 for the description of each of the 11 bands. The numbers on the left correspond to the bands of the molecular weight marker shown between the blots.
   b: 4-12% PAGE showing high molecular weight versican isoforms. Lane 1: rat brain. Lane 2 corresponds to normal breast sample digested with chondroitinase ABC. Bands 3 and 8 correspond to versican V2 isoform and GHAP (Glial Hyaluronate Binding Protein), respectively. The numbers on the left correspond to the bands of the molecular weight marker.
   c: 4-12% PAGE showing the migration level of recombinant versican V4 isoform (lane 2), tagged V4 isoform (lane 3), and V3 isoform (lane 4, initially lane 6 from the same blot) versus the migration level of versican entities in a representative breast tumor sample digested with chondroitinase ABC (lane 1). The molecular weight marker is shown to the left of the blot.

### DETAILED DESCRIPTION

Unless defined otherwise, all technical and scientific terms used herein have the same meaning as commonly understood by one of ordinary skill in the art. In any case, the meaning of a term should not be limited to less than would be commonly understood by one of ordinary skill. The examples are only illustrative and not limiting.

### Definitions

As used herein, the term "versican" refers to the versican gene, the RNA transcribed therefrom and the proteins encoded thereby. The general reference to 'versican' thus includes the alternatively spliced isoforms (V0, V1, V2, V3, V4) and the corresponding proteins. A "versican isoform" as used herein, refers to a nucleic acid variant (mRNA, cDNA) generated by alternative splicing, as well as to the protein encoded thereby. If only the protein (or, alternatively, only the gene) is intended, this is indicated throughout the specification by referring to the "versican protein" (or "versican gene" respectively).

The term versican most particularly encompasses human versican (GeneID 1462; Human Protein Reference Database 00340). The reference sequence for human versican mRNA is Accession No. NM_004385 (V0 isoform). Of note, the first exon and part of the 15^{th} exon of human versican are not part of the coding sequence. When referring to (human) versican nucleic acid, both forms containing these sequences (e.g. genomic DNA) and not containing these sequences (e.g. mature RNA) are envisaged. The same is true for versican protein, which may or may not contain the signal peptide encoded in exon 2. Exons 3-6 and 9-14 (or the amino acids they encode) are present in all complete versican isoforms, irrespective of the stage in transcription, translation or signal peptide processing.

Versican isoforms share a homologous globular N-terminal (G1), C-terminal (G3) and optionally glycosaminoglycan (GAG) binding regions. The different versican isoforms are characterised by the presence or absence of exons 7 (encoding the GAGα domain) and 8 (encoding the GAGβ domain). As used herein, the "V0 isoform of versican", also referred to as "V0 versican isoform", "V0 isoform" or "V0" (Accession No. for mRNA: NM_004385) contains both exons 7 and 8 (or, in the case of the protein V0 isoform, contains both the GAGα and GAGβ domains).

As used herein, the "V1 isoform of versican", also referred to as "V1 versican isoform", "V1 isoform" or "V1" (Accession No. for mRNA: X15998) contains exon 8 but lacks exon 7 (or, in the case of the protein V1 isoform, contains the GAGβ domain but lacks the GAGα domain).

As used herein, the "V2 isoform of versican", also referred to as "V2 versican isoform", "V2 isoform" or "V2" (Accession No. for mRNA U26555) contains exon 7 but lacks exon 8 (or, in the case of the protein V2 isoform, contains the GAGα domain, but lacks the GAGβ domain).

As used herein, the "V3 isoform of versican", also referred to as "V3 versican isoform", "V3 isoform" or "V3" (Accession No. for mRNA D32039) lacks both exons 7 and 8 (or, in the case of the protein V3 isoform, lacks both the GAGα and GAGβ domains).

As used herein, the "V4 isoform of versican", also referred to as "V4 versican isoform", "V4 isoform" or "V4" lacks exon 7 and contains part of exon 8, i.e. the first 1194 nucleotides of the 5262 nucleotides that comprise exon 8. These 1194 nucleotides are referred to herein as "exon 8a" (SEQ ID NO: 4). The protein V4 isoform thus lacks the GAGα domain and contains part of the GAGβ domain. The V4 isoform of exon is characterized in that it contains exon 8a, corresponding to SEQ ID NO: 4 directly flanked by exon 6 at its 5' end and by exon 9 at its 3' end. The junction of exons 8a and 9 represents a sequence that is specific to the V4 isoform, as it is not found in any of the other versican isoforms.

The terms "boundary region", "exon boundary" or "exon junction" as used herein when referring to two exons refers to a contiguous nucleic acid sequence spanning the junction between two adjacent exons in an alternatively spliced isoform, comprising at least three, more particularly six nucleotides of each of the adjacent exons, more in particular at least nine nucleotides of each of the adjacent exons, even more in particular at least twelve nucleotides of each of the adjacent exons.

As used herein, a (nucleic acid sequence or protein) "fragment" of the V4 isoform" is a nucleic acid sequence or protein fragment comprising a contiguous sequence corresponding to 80% or less of the complete versican V4 isoform.

As used herein a "V4-specific fragment" (of a nucleic acid sequence or protein) is a sequence comprising a sequence spanning the junction between exons 8a and 9 in the V4 isoform, or the peptide encoded thereby. A V4-specific fragment need not to contain an equal amount of nucleotides (or amino acids) of each of the adjacent exons, although such fragments are also envisaged. The length of a V4-specific nucleic acid fragment is particularly at least 30 nucleotides, at least 75 nucleotides, at least 90 nucleotides, at least 150 nucleotides or at least 300 nucleotides. The length of a V4-specific amino acid sequence is at least 10 amino acids, at least 25 amino acids, at least 30 amino acids, at least 50 amino acids or at least 100 amino acids.

As used herein, the term "mature transcript" refers to mature mRNA, i.e. mRNA that has been spliced and processed and is ready for translation.

The phrase "determining the presence and/or level of gene transcription" as used herein refers to the qualitative and/or quantitative determination of gene transcription at the nucleic acid (RNA, cDNA) or protein level. Determining the presence of gene transcription also encompasses determining the absence of gene transcription, i.e. the finding that no detectable transcription occurs. Similarly, determining the level of gene transcription also encompasses the determining of the absence of gene transcription, corresponding to a zero level. In particular embodiments the upregulation of gene transcription is determined (e.g. level of gene transcription in a test sample compared to control).

The "sequence identity" of two sequences as used herein relates to the number of positions with identical nucleotides or amino acids divided by the number of nucleotides or amino acids in the shorter of the sequences, when the two sequences are aligned. The alignment of two nucleotide sequences is particularly performed by the algorithm as described by Wilbur and Lipmann (1983) Proc. Natl. Acad. Sci. U.S.A. 80:726, using a window size of 20 nucleotides, a word length of 4 nucleotides, and a gap penalty of 4.

Two amino acids are considered as "similar" if they belong to one of the following groups GASTCP; VILM; YWF; DEQN; KHR. Thus, sequences having "sequence similarity" means that when the two protein sequences are aligned the number of positions with identical or similar nucleotides or amino acids divided by the number of nucleotides or amino acids in the shorter of the sequences, is higher than 80%, particularly at least 90%, even more particularly at least 95% and most particularly at least 99%, more specifically is 100%.

The present invention describes that the known versican isoforms are differentially expressed in human breast cancer tissues and adjacent normal counterparts. Versican overexpression in human breast cancer has been reported following immunohistochemical analyses. The mRNA and protein expression levels of different molecular weight versican isoforms were evaluated using quantitative RT-PCR and 1 D-PAGE. A new alternatively spliced versican isoform, referred to as "V4" has been identified. This new isoform is overexpressed in human breast cancer, making it useful as a screening tool.

One aspect of the present invention relates to nucleic acid molecules encoding the newly identified V4 isoform of Versican. A particular embodiment relates to isolated nucleic acid molecules encoding the V4 isoform of versican. This V4 isoform of versican is most easily defined by comparison to the V1 isoform of versican. The V4 isoform corresponds to the V1 isoform of versican except that exon 8 is replaced by exon 8a, which is an exon corresponding to the first 1194 nucleotides of exon 8. As a consequence, exon 8a is flanked at its 5' end by exon 6 and at its 3' end by exon 9 in the mature transcript of the V4 isoform. Accordingly the exon junction between exon 8a and exon 9 is specific for the V4 isoform and can be used to differentiate the V4 isoform from other versican isoforms.

In particular embodiments, nucleic acid molecules encoding the V4 isoform of versican are characterized in that they comprise a sequence having at least 80 % identity to SEQ ID NO:1, more in particular at least 90% identity, more particularly at least 95% identity, especially at least 98% identity, most particularly at least 99% identity to SEQ ID NO:1. A most particular embodiment provides a nucleic acid molecule encoding the V4 isoform of versican comprising a sequence according to SEQ ID NO: 1.

The invention further provides fragments of nucleic acid molecules encoding the V4 isoform of versican. More particularly, the invention provides V4-specific nucleic acid fragments. These fragments are characterized in that they comprise at least a sequence spanning the exon boundary between exons 8a and 9, which junction is characteristic to the V4 isoform. In particular embodiments, V4-specific nucleic acid fragments comprise a contiguous sequence spanning the junction between exon 8a and 9, comprising at least six nucleotides of each of the adjacent exons 8a and 9. In further embodiments the sequence spanning the exon 8a-exon 9 junction comprises between 12 and at least 18 nucleotides of the exons 8a-exon 9 junction, more particularly between 12 and 24 nucleotides thereof. Such V4-specific fragments need not contain an equal number of nucleotides from each exon.

According to particular embodiments fragments of the V4 isoform contain at least a sequence spanning the exon boundary between exons 8a and 9, whereby at least 60%, more particularly at least 70% of the sequence of the fragment corresponds to exon 8a. In further particular embodiments fragments of the V4 isoform contain at least a sequence spanning the exon boundary between exons 8a and 9, whereby at least 30%, more particularly at least 50%, very particularly at least 90%, most particularly at least 95% of exon 8a is included in the V4 specific fragment.

According to further embodiments, V4-specific fragments are provided which comprise at least 50 nucleotides, more particularly at least 75 nucleotides, even more particularly between 100 and 1000 nucleotides of the V4 isoform.

A further aspect of the invention relates to proteins and protein fragments encoded by nucleic acids encoding the V4 isoform as described above, also referred to as V4 isoform proteins. V4-specific proteins fragments are characterized by the presence of an amino acid sequence encoded by the exon 8a-exon 9 junction.

It has been found that versican isoforms, including the versican V4 isoform described above are differentially expressed in disease conditions. More particularly it has been found that the different versican isoforms are differentially expressed in tumour tissues compared to non-tumour tissue, and are thus of use in the diagnosis and therapy of cancer.

Accordingly, a further aspect of the invention relates to the methods of detecting cancer, more particularly tumour formation, which are based on determining the expression of one or more versican isoforms.

According to specific embodiments of this aspect, such methods comprise the step of determining in the tissue the presence and/or level of gene transcription of the one or more isoforms of versican, whereby the presence and/or level of gene transcription of the one or more isoforms of versican is indicative of the presence and/or status of a/the tumour. According to particular embodiments such methods comprise determining in the tissue the presence and/or level of gene transcription of one or more isoforms of versican, whereby the presence and/or level of gene transcription of the one or more isoforms of versican is indicative of the presence and/or status of a/the tumour. Accordingly, such methods most particularly comprise:
(a) determining in the tissue the presence and/or level of gene transcription of one or more isoforms of versican;
(b) determining the presence and/or status of said tumor based on the result from step (a).

In particular embodiments, the one or more isoforms of versican are selected from the group consisting of V0, V1, V2, V3 and the V4 isoform described herein. Particular embodiments of methods envisaged herein include determining one, two, three, four or all five isoforms, and potentially further isoforms to be identified.

Particular embodiments of this aspect of the invention relate to methods for determining the presence and/or status of a tumor in a tissue of a patient based on detection of gene transcription of at least the V4 isoform. According to specific embodiments of this aspect, such methods comprise the step of determining in the tissue the presence and/or level of gene transcription of the V4 isoform of versican, optionally combined with the determination of the presence and/or level of gene transcription of other isoforms of versican, whereby the presence and/or level of gene transcription of the V4 isoform (and optionally other isoforms) of versican is indicative of the presence and/or status of said tumour. Accordingly, such methods most particularly comprise:
(a) determining in the tissue the presence and/or level of gene transcription of at least the V4 isoform of versican;
(b) determining the presence and/or status of said tumor based on the result from step (a).

In particular embodiments, methods are provided which further encompass, determining the presence and/or level of gene transcription of other isoforms of versican, such as V0, V1, V2 and/or the V3 isoform.

Particular embodiments of methods described herein are methods that are carried out *in vitro,* i.e. on isolated tissue samples. A non-limiting list of examples of suitable tissue material includes tissue material is selected from the group consisting of body fluid, tissue biopsy material or autopsy material. Tissue material can be obtained by incisional biopsy, core biopsy or excisional biopsy. In particular embodiment, the tissue is obtained by needle aspiration biopsy.

Methods of the present invention relate to determining whether or not there is tumour formation in a sample. Accordingly, in particular embodiments, the tissue under investigation is a (suspected) tumour biopsy sample.

Methods according to the present invention may involve pre-treatment of the tissue material prior to performing the detection of versican isoform transcription. Typically samples on which the detection is performed are RNA extracts, tissue sections, ... etc.

Further embodiments of methods described herein are methods wherein detection is performed *in situ.* In these embodiments, the methods are performed on a tissue as present in the body. Methods of detection *in situ* include methods that make use of a labelled antibody or probe. The label is selectively localised at the binding site of the antibody or probe and may be visualised using appropriate detection methods (e.g. fluorescence in situ hybridisation (FISH), chromogenic in situ hybridisation, bright-field microscopy, confocal microscopy, coherence-gated microscopy, single- or multi-photon microscopy, endoluminal spectroscopy, magnetic resonance spectroscopy, Microelectromechanical systems (MEMS) technology). Labels that may be used include, but are not limited to, metal labels, enzymatic labels, fluorescent labels, luminescent labels, or combinations thereof. When performing in situ detection at the nucleic acid level, the hybridisation step may be followed by a signal amplification step to enhance the labelling. Such in situ detection is traditionally performed with long labelled probes. Alternatively, small circular probes may be used in combination with target primed rolling circle DNA synthesis. This technique has both been applied for DNA detection in situ, and for RNA targets (Stougaard et al., 2007).

In particular embodiments of methods described hereinabove, the step of determining in the tissue the presence and/or level of gene transcription of the V4 isoform of versican further comprises determining the presence and/or level of gene transcription of the V4 isoform of versican in a control tissue or control sample and comparing the presence and/or level of gene transcription of the V4 isoform of versican between sample and control tissue. The control tissue is typically a sample of tissue, more particularly a tissue of a similar or of identical nature as that of the test sample. In more particular embodiments the control is tissue obtained from the patient from which the test tissue originates. Alternatively, the level of gene transcription is compared to a standard tissue.

In particular embodiments of methods described herein, upregulation of gene transcription in the test tissue compared to control is indicative of tumor formation. Upregulation may be determined in a qualitative and/or quantitative way. Qualitative determination of upregulation involves determining whether or not there is upregulation in the test sample compared to control. Typically however, upregulation will be determined in a quantitative fashion, whereby the degree of upregulation is determined, by determining and comparing the level of gene transcription of the versican isoform in both test sample and control. The methods by which gene transcription is determined in methods of the present invention are not critical and include detection at the mRNA or protein level. Typical examples of methods to determine levels of gene transcription include, but are not limited to, quantitative RT-PCR, gel electrophoresis, mass spectrometry and (computer-assisted) quantification of proteins on Western blots. Where gene transcription of more than one versican isoform is assessed, this is typically performed by the same methods for all isoforms. However, methods involving detection of the different isoforms using different methods are also envisaged.

In particular embodiments of the present invention where upregulation is determined quantitatively, the methods involve determining whether, in the test tissue, the upregulation of gene transcription of a versican isoform compared to control is at least 10%, particularly at least 15%, more particularly at least 20%. In particular embodiments the methods involve determining an increase of expression of at least 25%, at least 30%, at least 40%, at least 50%, at least 75% or at least 100%.

According to particular embodiments of methods described hereinabove, the presence and/or level of gene transcription of one or more versican isoforms, more particularly of V4, is determined at the RNA level. More particularly one or more primers, primer sets or probes are used which are capable of generating a signal which is specific for a versican isoform. Such probes and primers or primer-sets are also referred to herein as "capable of specifically recognising a versican isoform".

According to particular embodiments, such probes or primers capable of specifically recognising a versican isoform are probes that hybridise to a sequence, which is specific for a versican isoform.

Accordingly a further aspect of the present invention provides probes, primers or primer sets which are specific for a versican isoform. More particularly, probes, primers and primer sets specific for the newly identified V4 isoform are provided. In particular embodiments such probes, primers and primer sets are capable of generating a signal specific for the V4 isoform based on their specific hybridisation with a sequence comprising the exon 8a-exon 9 junction.

According to alternative embodiments, primers sets capable of specifically recognising an isoform are primers sets which amplify a sequence which is specific for a versican isoform. Such specificity can be obtained based either on the size of the generated PCR fragment or on the sequence of the generated fragment (which can be specifically detected). Accordingly, in these embodiments, the primers themselves may hybridise to a sequence that is not specific to a versican isoform, but the amplified sequence is.

Accordingly, a further aspect of the present invention provides primer sets capable of amplifying a sequence specific for a versican isoform. More particularly primer sets capable of amplifying a sequence specific for the V4 isoform are provided. Particular embodiments thereof are primers which amplify a sequence between exons 6 and 9, as such primers when in contact with the V4 isoform will generate a specific fragment of ∼1200 nucleotides (corresponding to exon 8a), which is not generated when these primers are contacted with one of the other isoforms of versican.

According to particular embodiments of methods described hereinabove, the presence and/or level of gene transcription of one or more versican isoforms, such as the V4 isoform, is determined at the protein level. In particular embodiments ligands are used capable of specifically detecting each of the versican isoforms to be detected. Examples of such ligands are antibodies and antigen-binding fragments thereof. Additionally or alternatively, antibodies are used in combination with size-separation of the different versican proteins (or enzymatic fragments thereof). In the latter embodiments, one or more antibodies are used which recognise a sequence common to two or more of the versican isoforms to be detected. As the fragments generated are specific for each isoform, the antibody need not be specific.

In particular embodiments, the V4 isoform is detected using an anti-versican antibody or an antibody specific for the V4 isoform of versican. Both polyclonal and monoclonal anti-versican antibodies are well known in the art and include for instance anti-Vc, mAb 5D5, mAb 12C5 and mAb 2B1 antibodies (Cattaruzza et al., 2002). According to a particular embodiment, the anti-versican antibody used is 12c5.

Specific antibodies for the different versican isoforms including the V4 versican isoform can be generated using methods well known in the art. An antibody specific for an isoform of versican is an antibody that binds to an epitope specific for the isoform.

A further aspect of the present invention relates to versican-isoform specific ligands. Most particularly such ligands are antibodies or antigen-binding fragments thereof. More particularly, the present invention provides antibodies specifically recognising the V4 isoform. It is demonstrated herein that the V4 isoform of versican contains the exon 8a-exon 9 junction, which is specific for the V4 isoform. Accordingly, particular embodiments of versican V4 isoform antibodies are antibodies specifically recognising the exon 8a-exon 9 boundary. Such antibodies are generating by immunising a host with either the complete V4 isoform protein (and selecting the V4 specific antibodies generated) or by immunising with a peptide thereof comprising the exon 8a-exon 9 junction.

Antibodies can be prepared using standard techniques. To prepare polyclonal antibodies or "antisera," an animal is inoculated with an antigen that is an isolated and purified polypeptide (here versican isoform protein or a fragment thereof), and immunoglobulins are recovered from a fluid, such as blood serum, that contains the immunoglobulins, after the animal has had an immune response. For inoculation, the antigen is preferably bound to a carrier peptide and emulsified using a biologically suitable emulsifying agent, such as Freud's incomplete adjuvant. A variety of mammalian or avian host organisms may be used to prepare polyclonal antibodies. Following immunisation, Ig is purified from the immunised bird or mammal, e.g., goat, rabbit, mouse, rat, or donkey and the like. For certain applications, it is preferable to obtain a composition in which the antibodies are essentially free of antibodies that do not react with the immunogen. This composition is composed virtually entirely of the high titer, monospecific, purified polyclonal antibodies to the antigen. Antibodies can be purified by affinity chromatography. Purification of antibodies by affinity chromatography is generally known to those skilled in the art (see, for example, U.S. Patent No. 4,533,630). Briefly, the purified antibody is contacted with the purified polypeptide, or a peptide fragment thereof, bound to a solid support for a sufficient time and under appropriate conditions for the antibody to bind to the polypeptide or peptide. Such time and conditions are readily determinable by those skilled in the art The unbound, unreacted antibody is then removed, such as by washing. The bound antibody is then recovered from the column by eluting the antibodies, so as to yield purified, monospecific polyclonal antibodies.

Monoclonal antibodies can be also prepared, using known hybridoma cell culture techniques. In general, this method involves preparing an antibody-producing fused cell line, e.g., of primary spleen cells fused with a compatible continuous line of myeloma cells, and growing the fused cells either in mass culture or in an animal species, such as a murine species, from which the myeloma cell line used was derived or is compatible. Such antibodies offer many advantages in comparison to those produced by inoculation of animals, as they are highly specific and sensitive and relatively "pure" immunochemically. Immunologically active fragments of the present antibodies are also envisaged, e.g., the F(ab) fragment, scFv's, as are partially humanised monoclonal antibodies.

It will be understood by those skilled in the art that a monoclonal antibody may be subjected to the techniques of recombinant DNA technology to produce other derivative antibodies, humanised or chimeric molecules or antibody fragments which retain the specificity of the original monoclonal antibody. Such techniques may involve combining DNA encoding the immunoglobulin variable region, or the complementarity determining regions (CDRs), of the monoclonal antibody with DNA coding the constant regions, or constant regions plus framework regions, of a different immunoglobulin, for example, to convert a mouse-derived monoclonal antibody into one having largely human immunoglobulin characteristics.

The antibodies generated are used in the context of the present invention for detecting the presence or determining the gene transcription level of one or more versican isoforms, more particularly determining the gene transcription level of isoform V4 in a sample. Typically, the antibodies are contacted with the sample for a period of time and under conditions sufficient for antibodies to bind to the polypeptide so as to form a binary complex between at least a portion of the antibodies and the polypeptide. The presence of the complex may then be detected by one of a variety of antibody-based detection methods, such as, but not limited to a sandwich assay, ELISA assay, radiolabelling assay or other known techniques for antibody-antigen detection. Incubation times, conditions and reaction media can be readily determined by persons skilled in the art. For example, where appropriate, cells can be lysed to yield an extract which comprises cellular proteins. Alternatively, intact cells are permeabilised in a manner which permits macromolecules, i.e., antibodies, to enter the cell. The antibodies are then incubated with the protein extract, e.g., in a Western blot, or permeabilised cells, e.g., prior to flow cytometry, so as to form a complex. The presence or amount of the complex is then determined or detected.

The antibodies of the invention may also be coupled to an insoluble or soluble substrate. Soluble substrates include proteins such as bovine serum albumin. Preferably, the antibodies are bound to an insoluble substrate, i.e., a solid support. The antibodies are bound to the support in an amount and manner that allows the antibodies to bind the polypeptide (ligand). The amount of the antibodies used relative to a given substrate depends upon the particular antibody being used, the particular substrate, and the binding efficiency of the antibody to the ligand. The antibodies may be bound to the substrate in any suitable manner. Covalent, noncovalent, or ionic binding may be used. Covalent bonding can be accomplished by attaching the antibodies to reactive groups on the substrate directly or through a linking moiety. The solid support may be any insoluble material to which the antibodies can be bound and which may be conveniently used in an assay of the invention. Such solid supports include permeable and semipermeable membranes, glass beads, plastic beads, latex beads, plastic microtiter wells or tubes, agarose or dextran particles, sepharose, and diatomaceous earth. Alternatively, the antibodies may be bound to any porous or liquid permeable material, such as a fibrous (paper, felt etc.) strip or sheet, or a screen or net. A binder may be used as long as it does not interfere with the ability of the antibodies to bind the ligands.

Further embodiments of methods involving the specific detection of one or more isoforms of versican involve methods that make use of molecules capable of generating versican-isoform specific RNA or protein fragments. These methods include methods that involve specific enzymes, which cleave RNA or protein. As each of the versican isoforms comprises a different combination of exons, specific fragments can be generated for each of the isoforms.

Accordingly, methods are provided herein which make use of reagents capable at the RNA or protein level, of specifically detecting a versican isoform. In these methods, the specific reagents allow a qualitative and optionally quantitative detection of the level of gene transcription of one or more of the versican isoforms. In particular embodiments the isoform-specific reagents are labelled. The nature of the label is not critical and will be determined by the method in which the reagent is used. Typical examples of labels include, but are not limited to fluorescent labels, luminescent labels, Raman labels, radioactive labels, and enzymatic labels. Alternatively, methods are envisaged wherein one or more reagents capable of specifically recognising a versican isoform are used together with secondary reagents which ensure the signal (e.g. secondary labelled antibodies, labelled probes etc.).

A further aspect of the invention relates to kits for performing methods such as those described hereinabove, i.e. kits which comprise reagents capable of specifically recognising one or more versican isoforms. Accordingly, kits according to the present invention comprise at least one of the following:
- one or more oligonucleotides or sets of oligonucleotides capable of specifically recognising an isoform of versican; and/or
- one or more antibodies capable of specifically detecting a versican isoform.

According to particular embodiments, kits suitable for detecting the V4 isoform of versican are provided. In particular embodiments such kits comprise:
- one or more oligonucleotides or sets of oligonucleotides capable of specifically recognising a V4 isoform of versican; and/or
- one or more antibodies capable of specifically detecting a V4 versican isoform.

According to a further specific embodiment, kits are provided which comprise at least one oligonucleotide or set of oligonucleotides capable of specific recognition of the V4 isoform of versican by specifically hybridising to the exon boundary between exons 8a and 9. According to further specific embodiments kits are provided which comprise an antibody capable of specifically detecting a protein comprising the sequence encoded by the exon boundary between exons 8a and exon 9.

In particular embodiments kits are provided as described above for detecting the presence and/or status of a tumour in a tissue.

Particular embodiments of methods described herein allow determination of the status of tumour in a tissue.

In particular embodiments, tumours are graded by four degrees of severity as recommended by the American Joint Commission on Cancer: grades 1, 2, 3, and 4. Wherein Grade 1 tumours are well-differentiated or low-grade tumours, Grade 2 tumours are moderately well-differentiated (intermediate grade tumours), Grade 3 are poorly differentiated high grade tumours and Grade 4 tumours are undifferentiated high-grade tumours. Grade 3 and grade 4 are the most aggressive in behaviour. This method is most common for tumours such as breast tumours.

Some cancers also have special grading systems. For example, pathologists use the Gleason system to describe the degree of differentiation of prostate cancer cells. The Gleason system uses scores ranging from Grade 2 to Grade 10. Lower Gleason scores describe well-differentiated, less aggressive tumours. Higher scores describe poorly-differentiated, more aggressive tumours.

It has moreover been found that the amount of glycosaminoglycan (GAG) attached to a versican isoform correlates with the status of the tumour. More particularly, for the V4 isoform, a correlation between the amount of GAG present on V4 in a tumour tissue and the status of the tumour has been noted.

Accordingly, in particular embodiments of the methods for determining the presence and/or status of a tumour described herein, the amount of GAG attached GAG to one or more versican isoform proteins is determined. In the methods described herein, a higher amount of GAG is indicative of a higher grading of the tumour. Particular embodiments of methods described herein involve both a qualitative and/or quantitative detection of one or more isoforms of versican and an identification of the amount of GAG attached to the one or more versican isoforms. In particular embodiments a qualitative and/or quantitative detection of the V4 isoform of versican and an identification of the amount of GAG attached to the V4 isoform is performed.

Methods for determining the amount of GAG present on a protein are known in the art. For instance, it can be determined by assaying the sensitivity of the protein to chondroitinase ABC, as described in the examples herein.

The present invention describes that the transcription of specific isoforms of versican is correlated with disease. More particularly it has been found that the transcription level of versican isoforms, more particularly of the V4 isoform of versican is correlated with the presence (and status) of tumours, more particularly the presence (and status) of a tumour of the breast. Accordingly, methods described herein are of particular interest in the diagnosis of breast cancer.

Accordingly, in particular embodiments of the invention, methods are provided for determining the presence and/or status of breast cancer in breast tissue of a patient, comprising the steps specifically determining in said tissue the presence and/or level of gene transcription of at least one isoform of versican selected from the group consisting of the V0 isoform, the V1 isoform, the V2 isoform, the V3 isoform, and the V4 isoform, wherein presence and/or level of gene transcription of the one or more isoforms of versican is indicative of the presence and/or status of the tumour.

A further aspect of the present invention relates to recombinant expression of the V4 isoform of versican. More particularly, recombinant expression of the isolated nucleic acids encoding V4 is envisaged. According to particular embodiments, a vector is provided comprising a nucleic acid molecule having a nucleic acid sequence encoding the V4 isoform of versican, or a V4-specific fragment thereof.

According to further particular embodiments, vectors are provided which comprises a nucleic acid molecule having a nucleic acid sequence encoding the V4 isoform of versican, i.e. encoding the complete isoform. In particular embodiments, vectors are provided which comprise nucleic acid molecules comprising a sequence having at least 80 % sequence identity with SEQ ID NO:1, more in particular at least 90% sequence identity, even more in particular at least 95% identity, yet even more in particular at least 98% identity, most in particular at least 99% identity to SEQ ID NO:1. In further particular embodiments, the nucleic acid sequence is SEQ ID NO:1.

A "vector" as used herein is a polynucleic acid molecule, which allows expression of a nucleic acid molecule integrated therein in a suitable host cell. To this end, a vector will typically comprise a promoter sequence operably combined with the nucleic acid molecule to be expressed, and possibly other elements (e.g. serving a regulatory or structural function). The polynucleic acid molecule (and the nucleic acid molecule integrated therein) may be circular or linear, double-stranded or single-stranded. The promoter used in the vector may be strong or weak, constitutive or inducible.

Other sequences functional in the host cells, such as introns, enhancers, polyadenylation sequences and the like, may also be a part of the vector. Such elements may or may not be necessary for the function of the DNA encoding the V4 isoform, but may provide improved expression of the DNA by affecting transcription, stability of the mRNA, or the like. As the DNA sequence between the transcription initiation site and the start of the coding sequence, i.e., the untranslated leader sequence, can influence gene expression, one may also wish to employ a particular leader sequence. Preferred leader sequences are contemplated to include those which include sequences predicted to direct optimum expression of the attached V4 gene, i.e., to include a preferred consensus leader sequence which may increase or maintain mRNA stability and prevent inappropriate initiation of translation. The choice of such sequences will be known to those of skill in the art. All of these various sequences may be operably linked although they are not necessarily directly linked.

The vector to be introduced into the host cells may also generally contain either a selectable marker gene or a reporter gene, or both, to facilitate identification and selection of transformed cells from the population of cells sought to be transformed. Alternatively, the selectable marker may be carried on a separate piece of DNA and used in a co-transformation procedure. Both selectable markers and reporter genes may be flanked with appropriate regulatory sequences to enable expression in the host cells. Useful selectable markers are well known in the art and include, for example, antibiotic and herbicide-resistance genes, such as *neo*, *hpt, dhfr, bar, aroA, dapA* and the like. See also, the genes listed in Table 1 of Lundquist et al. (U.S. Patent No. 5,848,956).

The vector may also comprise one or more reporter genes. Reporter genes are used for identifying potentially transformed cells and for evaluating the functionality of regulatory sequences. Reporter genes which encode for easily assayable proteins are well known in the art. In general, a reporter gene is a gene which is not present in or expressed by the recipient organism or tissue and which encodes a protein whose expression is manifested by some easily detectable property, e.g., enzymatic activity. Genes particularly envisaged include the chloramphenicol acetyl transferase gene (cat) of E. coli, the beta-glucuronidase gene (GUS) of E. coli, and the luciferase gene from firefly Photinus pyralis. Expression of the reporter gene is assayed at a suitable time after the DNA has been introduced into the recipient cells.

Plasmid vectors can include additional DNA sequences that provide for easy selection, amplification, and transformation of the expression cassette in prokaryotic and eukaryotic cells, e.g., pUC-derived vectors such as pUC8, pUC9, pUC18, pUC19, pUC23, pUCI 19, and pUC120, pSK-derived vectors, pGEM-derived vectors, pSP-derived vectors, or pBS-derived vectors. The additional DNA sequences include origins of replication to provide for autonomous replication of the vector, selectable marker genes, preferably encoding antibiotic or herbicide resistance, unique multiple cloning sites providing for multiple sites to insert DNA sequences or genes encoded in the expression cassette, and sequences that enhance transformation of prokaryotic and eukaryotic cells. Viral vectors are also useful for expression of V4 molecules (polynucleic acid or polypeptide) in eukaryotic and prokaryotic cells. Viral vectors useful in the present invention include, but are not limited to, adenoviral, AAV, LXSN, or baculoviral vectors.

Further particular embodiments of the methods for recombinant expression of the V4 isoform or fragments thereof, including V4-specific fragments include methods comprising the steps of:
- providing a host cell;
- transforming the host cell with an isolated and purified nucleic acid molecule having a nucleic acid sequence encoding the V4 isoform of versican or a fragment thereof, more particularly a fragment comprising the exon boundary between exons 8a and 9;
- culturing the host cell so that the host cell expresses the V4 isoform of versican or the V4-specific fragment thereof.

Typically, the nucleic acid molecule having a nucleic acid sequence encoding the V4 isoform of versican is integrated in a vector prior to transformation of the host cell with the nucleic acid molecule. Host cells can be any suitable mammalian, bacterial, plant, yeast or insect cell (e.g., baculoviral expression systems). Suitability may e.g. depend on whether the cells can be cultured, whether they can be transformed or transfected, whether the recombinantly produced protein can be easily recovered. Suitable host cells are well known in the art, and the nature of the host cell is not critical to the invention. According to particular embodiments, the host cell is a eukaryotic cell. According to further particular embodiments, the host cell is a mammalian cell. According to yet further particular embodiments, the host cell is a murine cell. According to alternative particular embodiments, the host cell is a human cell.

Methods for introducing a DNA into a host cell are known to the skilled person and include, but are not limited to, physical methods such as calcium phosphate precipitation, lipofection, particle bombardment, microinjection, electroporation, and the like.

Biological methods for introducing a DNA of interest into a host cell are known to the skilled person and include, but are not limited to, the use of DNA and RNA viral vectors. Viral vectors, and especially retroviral vectors, have become the most widely used method for inserting genes into mammalian, e.g., human cells. Other viral vectors can be derived from poxviruses, herpes simplex virus I, adenoviruses and adenoassociated viruses, and the like.

Host cells are typically produced by transfection with a DNA sequence in a plasmid expression vector, a viral expression vector, or possibly as an isolated linear DNA sequence.

To confirm the presence of the preselected DNA sequence in the host cell, a variety of assays may be performed. Such assays include, for example, "molecular biological" assays well known to those of skill in the art, such as Southern and Northern blotting, RT-PCR and PCR, and "biochemical" assays, such as detecting the presence or absence of a particular polypeptide, e.g., by immunological means (ELISAs and Western blots). To detect and quantitate RNA produced from introduced preselected DNA segments, RT-PCR may be employed. In this application of PCR, it is first necessary to reverse transcribe RNA into DNA, using enzymes such as reverse transcriptase, and then amplify the DNA using conventional PCR techniques. Further information about the nature of the RNA product may be obtained by Northern blotting. This technique demonstrates the presence of an RNA species and gives information about the integrity of that RNA. The presence or absence of an RNA species can also be determined using dot or slot blot Northern hybridisations. These techniques are modifications of Northern blotting and demonstrate the presence or absence of an RNA species. Southern blotting and PCR may be used to detect the preselected DNA segment in question. Expression of the DNA segment may be evaluated by specifically identifying the peptide products of the introduced DNA sequences or evaluating the phenotypic changes brought about by the expression of the introduced DNA segment in the host cell.

A further aspect of the invention provides isolated V4 proteins or peptides which are amino acid sequences comprising a sequence which is encoded by a nucleotide sequence comprising the exon boundary between exons 8a and 9. In particular embodiments the V4 isoform proteins or peptides comprise at least 10 amino acids, more particularly at least 30 amino acids.

In particular embodiments, V4 proteins or peptides are characterized in that they comprise a sequence encoded by a sequence having at least 80 % identity to SEQ ID NO:1 or a fragment thereof comprising the boundary between exons 8a and 9. Particular embodiments relate to proteins and peptides wherein the sequence comprising the boundary between exons 8a and 9 is has at least 70% sequence identity, more particularly at least 80% sequence identity, most particularly at least 90% sequence identity with SEQ ID NO:2. More particular embodiments relate to V4 isoform proteins or peptides which have at least 95% identity, especially at least 98% identity, most particularly at least 99% identity to SEQ ID NO: 2. A most particular embodiment provides a V4 isoform of versican or fragment thereof comprising SEQ ID NO: 2 or a fragment thereof.

In particular embodiments the V4 isoform peptide or protein is a recombinant protein or peptide. Alternatively, the peptide or protein is isolated from natural sources, such as, but not limited to breast cancer tissue.

It has been found that versican isoform transcription is associated with disease, more particularly with the presence and status of tumours. More particularly, it has been found that tumour tissue is characterized by an increase in expression of one or more versican isoforms. Accordingly it is envisaged that compounds capable of modifying versican isoform transcription may be of therapeutic benefit.

Accordingly, yet a further aspect of the present invention provides methods for screening agents capable of modulating the transcription of one or more forms of versican. More particularly, methods of identifying agents capable of modulating V4 isoform expression are envisaged. Such methods typically involve monitoring the effect of compounds on transcription of a V4 isoform in a host cell. According to particular embodiments, such methods comprise the steps of:
(a) providing a host cell comprising a nucleic acid molecule encoding the V4 isoform of versican
(b) contacting the host cell with an agent; and
(c) determining whether the agent modulates the transcription of the V4 isoform in the host cell.

According to a particular embodiment, modulating the transcription of the V4 isoform encompasses modulating the translation of the V4 isoform in the host cell. Either way, the amount of V4 in the host cell is modulated by the agent. Thus, agents modulating the transcription of the V4 isoform may either be agents modulating only transcription of the V4 isoform, agents modulating only translation of the V4 isoform, agents modulating only the amount of V4 isoform in the cell (e.g. post-translational), or agents modulating more than one of these processes, up to all of these processes.

An "agent" as used herein may be any molecule or compound which may be of interest and which can be brought into contact with the host cell. In particular, the term agent includes small molecules (i.e. molecules smaller than 20 kDa, more in particular smaller than 5 kDa), antibodies, polynucleic acids (e.g. antisense, ribozymes) and polypeptides.

Typically, screening is performed with a read-out at the protein level, i.e. determining whether the amount of versican V4 protein is affected. However, it is also envisaged to screen for compounds or agents with a read-out at the gene transcription level, thus determining whether the amount of V4 mRNA changes upon contacting the host cell with the agent.

Modulating the amount of the V4 isoform comprises determining any change in the level of V4 transcription after contact with the agent. Both up-and downregulation of V4 can be assessed in this way, allowing identification both of molecules increasing or enhancing the production of V4 and of molecules inhibiting the production of the V4 versican isoforms. These may be useful in different applications.

As detailed above, it has been determined that expression of different versican isoforms is correlated with tumour formation. Accordingly, a further aspect of the invention provides methods and tools are which reduce the level of transcription of one or more versican isoforms.

In particular embodiments, methods and tools are provided which reduce the level of transcription of the V4 isoform.

In further particular embodiments, inhibitors of V4 isoform expression are provided as well as methods involving their use. Such inhibitors can act either at the DNA, RNA or protein level of V4 expression and include, but are not limited to inhibitory V4-specific antibodies, V4-specific small molecule inhibitors or V4-specific antisense nucleic acid molecules. A "small molecule" may be defined as a molecule smaller than 20 kDa, smaller than 15 kDa, smaller than 10 kDa, smaller than 5 kDa, smaller than 3 kDa, smaller than 2 kDa or smaller than 1 kDa. According to specific embodiments of the invention V4-specific inhibitors are provided which are directed against the nucleic acid sequence encoding the exon 8a-exon 9 boundary or the corresponding protein sequence; such inhibitors are typically specific for the V4 isoform.

Versican proteins have been identified to interact with different molecules including but not limited to hyaluronan, members of the Tenascin family and chemokines. Accordingly, in particular embodiments inhibitors of the V4 isoform are molecules which interfere with one or more of the interactions of V4 with other proteins and peptides.

Accordingly, yet a further aspect of the invention provides the use of a versican inhibitor in the treatment and/or prevention of tumour formation. More particularly the present invention provides methods for treating and preventing tumour formation in a patient, the methods comprising:
- providing an inhibitor of the V4 isoform of versican
- administering the inhibitor to the patient so as to reduce or prevent tumour formation.

According to particular embodiments, the inhibitor of the V4 isoform is selected from the group consisting of: an inhibitory antibody specific for the V4 isoform of versican, a small molecule inhibitor of the V4 isoform of versican or an antisense nucleic acid molecule directed against the V4 isoform of versican.

According to further particular embodiments, methods are provided for the treatment of breast cancer. Typically, this comprises the use of an inhibitor of the V4 isoform of versican for the manufacture of a medicament for the treatment of breast cancer.

In a further embodiment, methods of treating a tumour in a patient are provided, comprising administering to the patient a therapeutically effective amount of an inhibitor of the V4 isoform of versican, selected from an inhibitory antibody specific for the V4 isoform of versican, a small molecule inhibitor of the V4 isoform of versican or an antisense nucleic acid molecule directed against the V4 isoform of versican. In a more particular embodiment, the methods further comprise the step of evaluating tumour size after administering of the V4 inhibitor.

According to a particular embodiment of the methods of treatment, the tumour to be treated is a breast tumour or breast cancer.

In a further aspect of the invention, the specific expression of the V4 isoform in a subset of cells is used in drug-targeting. According to this aspect of the invention ligands which bind with high affinity and specificity to V4 are used to ensure the delivery of therapeutic or diagnostic agents.

Accordingly, this aspect of this aspect of the invention makes use of ligands specifically binding to the V4 isoform of versican. In particular embodiments the ligand is a molecule which recognizes V4 based on antigen-antibody binding, i.e. an antibody an antibody fragment or derivative thereof which binds specifically to V4. Antibodies directed against the V4 isoform of versican are discribed hereinabove, however the antibodies used on the context of this aspect of the invention need not be inhibitory antibodies. Other high affinity ligands suitable for use in targeting include but are not limited to high-affinity binding peptides (Colleins et al. 2001), aptamers (Nimjee et al., 2005) and synthetic organic molecules (Melkko et al., 2004). Methods and ligands for targeting drugs and therapeutics are described e.g. in US patents Nos. US 3,927,193 and US 4,331,647. To counter the disadvantages of direct targeting methods, pretargeting methods have also been discribed (e.g. US Pat. No. 4,863,713; US Pat. No. 5,525,338) as well as alternatives which make use of bi- or tri-speficic targeting agents (e.g. US patent application US2004/0241158).

According to this aspect of the invention a drug or a diagnostic agent is delivered to the site where the ligand binds. The mechanism of delivery can be either directly, i.e. by binding of the drug to the V4-binding ligand, or indirectly, by providing a linker which will bind and/or activate the drug or therapeutic agent to the location where V4 is expressed. Methods for coupling ligands to a drug or a diagnostic agent are known in the art.

According to a particular embodiment, methods are provided using V4 for drug-targeting, i.e. for the delivery of one or more bioactive agents. Different types of bioactive agents are envisaged such as, but not limited to drugs, cytokines, toxins, procoagulant factors, radionuclides, photosensitizers etc.. In specific embodiments, the drug is an anti-tumor drug. Examples of suitable anti-cancer drugs include but are not limited to small molecules such as adriamycin, taxol, cis-platin, mytomycin-C, daunomycin and 5-fluorouracil.

In further particular embodiments the methods of this aspect of the invention are used for the treatment and/or prevention of tumour formation. More specifically this aspect of the invention envisages the reduction or inhibition of growth of tumors expressing the V4 isoform of versican. According to a particular embodiment of the methods of treatment, the tumor to be treated is a breast tumor or breast cancer. Accordingly the invention provides method for targeting an anti-cancer drug to a breast tumor in a patient, which comprise the steps of linking the anti-cancer drug to a ligand specific for the V4 isoform of versican so as to form and administering the ligand so obtained to the patient. In particular embodiments the targeted drug is administered intravenously.

According to particular embodiments, methods are provided using V4 for targeting of a diagnostic agent. Non-limiting examples of diagnostic agents include a photoactive agent or dye, a radionuclide, a radioopaque material, a contrast agent, a fluorescent compound, an enhancing agent for magnetic resonance imaging (MRI) and combinations thereof.

Accordingly the present invention provides ligands which bind specifically to the V4 isoform of versican and which are linked to or labelled with a bioactive or diagnostic agent. The invention further provides methods of therapy and diagnosis which make use of such labeled V4-specific ligands.

In a further aspect of the invention, the V4 isoform of versican and V4 specific fragments thereof are provided for use as a medicament. Both the protein V4 isoform and the nucleic acid isoform may be used to this end. In a particular embodiment, the V4 isoform of versican is provided for use in the treatment of breast cancer. Typically, this may comprise the use of the V4 isoform of versican for the manufacture of a medicament for the treatment of breast cancer.

In a particular embodiment, methods of treating a tumor in a patient are provided, comprising administering to the patient a therapeutically effective amount of versican V4 isoform. Typically, the protein will be used in such methods of treatment. However, administering the versican V4 nucleic acid isoform is envisaged as well, e.g. in gene therapy. In a more particular embodiment, the methods further comprise the step of evaluating tumor size after administering of V4.

According to a particular embodiment of the methods of treatment, the tumor to be treated is a breast tumor or breast cancer.

The term "therapeutically effective amount" as used herein refers to an amount of the peptide of the invention or derivative thereof, which produces the desired therapeutic or preventive effect in a patient. For example, in reference to a disease or disorder, it is the amount which reduces to some extent one or more symptoms of the disease or disorder, and more particularly returns to normal, either partially or completely, the physiological or biochemical parameters associated with or causative of the disease or disorder. According to one particular embodiment of the present invention, the therapeutically effective amount is the amount of the peptide of the invention or derivative thereof, which will lead to an improvement or restoration of the normal physiological situation. For instance, when used to therapeutically treat a mammal affected by an immune disorder, it is a daily amount peptide/kg body weight of the said mammal. Although the dosage and mode of administration will depend on the individual, generally, the medicament is administered so that the compound, agent, protein, polypeptide, peptide of the present invention is given at a dose between 1 µg/kg and 10 mg/kg, more preferably between 10 µg/kg and 5 mg/kg, most preferably between 0.1 and 2 mg/kg. Preferably, it is given as a bolus dose. Continuous infusion may also be used and includes continuous subcutaneous delivery via an osmotic minipump. If so, the medicament may be infused at a dose between 5 and 20 µg/kg/minute, more preferably between 7 and 15 µg/kg/minute.

Alternatively, where the administration is through gene-therapy, the amount of naked DNA or viral vectors is adjusted to ensure the local production of the relevant dosage of the peptide of the invention, derivative or homologue thereof. Thus, where the administration of one or more peptides according to the invention is ensured through gene transfer (i.e. the administration of a nucleic acid which ensures expression of peptides according to the invention in vivo upon administration), the appropriate dosage of the nucleic acid can be determined based on the amount of peptide expressed as a result of the nucleic acid, such as e.g. by determining the concentration of peptide in the blood after administration. Thus, in a particular embodiment, peptides are administered through the use of polynucleotides encoding said peptides, whether in an expression vector or not, and thus the present invention also relates to gene therapy methods. Another particular embodiment relates to the use of methods to induce a local overexpression of the peptides of the invention for the treatment or prevention of certain disorders.

Versican V4, or a versican V4 inhibitor may be administered by any route appropriate to the condition to be treated and appropriate for the compounds to be administered. Possible routes include, but are not limited to, regional, systemic, oral (solid form or inhalation), rectal, nasal, topical (including ocular, buccal and sublingual), vaginal and parenteral (including subcutaneous, intramuscular, intravenous, intradermal, intraarterial, intrathecal and epidural). The preferred route of administration may vary with for example the condition of the recipient or with the diseases to be treated. Typically, versican V4 or a versican V4 inhibitor will be administered in a pharmaceutical composition or formulation, together with pharmaceutically acceptable carriers. As described herein, the carrier(s) optimally are "acceptable" in the sense of being compatible with the other ingredients of the formulation and not deleterious to the recipient thereof. The formulations include those suitable for oral, rectal, nasal, topical (including buccal and sublingual), vaginal or parenteral (including subcutaneous, intramuscular, intravenous, intradermal, intraarterial, intrathecal and epidural) administration. The formulations may conveniently be presented in unit dosage form and may be prepared by any of the methods well known in the art of pharmacy. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product. Formulations of the present invention suitable for oral administration may be presented as discrete units such as capsules, cachets or tablets each containing a predetermined amount of the active ingredient; as a powder or granules; as solution or a suspension in an aqueous liquid or a non-aqueous liquid; or as an oil-in-water liquid emulsion or a water-in-oil liquid emulsion. The active ingredient may also be presented as a bolus, electuary or paste. A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, preservative, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

For local treatments for example on the skin, such as of the joint, the formulations are optionally applied as a topical ointment or cream containing the active ingredient(s) in an amount of, for example, 0.075 to 20% w/w (including active ingredient(s) in a range between 0.1% and 20% in increments of 0.1% w/w such as 0.6% w/w, 0.7% w/w, etc), particularly 0.2 to 15% w/w and more particularly 0.5 to 10% w/w. When formulated in an ointment, the active ingredients may be employed with either a paraffinic or a water-miscible ointment base. Alternatively, the active ingredients may be formulated in a cream with an oil-in-water cream base. If desired, the aqueous phase of the cream base may include, for example, at least 30% w/w of a polyhydric alcohol, i.e. an alcohol having two or more hydroxyl groups such as propylene glycol, butane 1,3-diol, mannitol, sorbitol, glycerol and polyethylene glycol (including PEG400) and mixtures thereof. The topical formulations may desirably include a compound that enhances absorption or penetration of the active ingredient through the skin or other affected areas. Examples of such dermal penetration enhancers include dimethylsulfoxide and related analogues. The oily phase of the emulsions of this invention may be constituted from known ingredients in a known manner. While the phase may comprise merely an emulsifier (otherwise known as an emulgent), it desirably comprises a mixture of at least one emulsifier with a fat or an oil or with both a fat and an oil. Optionally, a hydrophilic emulsifier is included together with a lipophilic emulsifier that acts as a stabiliser, typically by including both an oil and a fat. Together, the emulsifier(s) with or without stabiliser(s) make up the so-called emulsifying wax, and the wax together with the oil and fat make up the so-called emulsifying ointment base which forms the oily dispersed phase of the cream formulations.
The choice of suitable oils or fats for the formulation is based on achieving the desired cosmetic properties, since the solubility of the active compound in most oils likely to be used in pharmaceutical emulsion formulations is very low. Thus, the cream should optionally be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, and particularly butyl stearate, 2-ethylhexyl palmitate or a blend of branched chain esters known as Crodamol CAP may be used. These may be used alone or in combination depending on the properties required. Alternatively, high melting point lipids such as white soft paraffin and/or liquid paraffin or other mineral oils can be used. Formulations suitable for topical administration to the eye also include eye drops wherein the active ingredient is dissolved or suspended in a suitable carrier, especially an aqueous solvent for the active ingredient. The active ingredient is optionally present in such formulations in a concentration of 0.5 to 20%, advantageously 0.5 to 10% particularly about 1.5% w/w. Formulations suitable for topical administration in the mouth include lozenges comprising the active ingredient in a flavoured basis, usually sucrose and acacia or tragacanth; pastilles comprising the active ingredient in an inert basis such as gelatin and glycerine, or sucrose and acacia; and mouthwashes comprising the active ingredient in a suitable liquid carrier. Formulations for rectal administration may be presented as a suppository with a suitable base comprising for example cocoa butter or a salicylate. Formulations suitable for nasal administration wherein the carrier is a solid include a coarse powder having a particle size for example in the range 20 to 500 microns (including particle sizes in a range between 20 and 500 microns in increments of 5 microns such as 30 microns, 35 microns, etc), which is administered in the manner in which snuff is taken, i.e. by rapid inhalation through the nasal passage from a container of the powder held close up to the nose. Suitable formulations wherein the carrier is a liquid, for administration as for example a nasal spray or as nasal drops, include aqueous or oily solutions of the active ingredient. Formulations suitable for aerosol administration may be prepared according to conventional methods and may be delivered with other therapeutic agents. Formulations suitable for vaginal administration may be presented as pessaries, tampons, creams, gels, pastes, foams or spray formulations containing in addition to the active ingredient such carriers as are known in the art to be appropriate. Formulations suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets of the kind previously described.

Typical unit dosage formulations are those containing a daily dose or unit daily sub-dose, as herein above recited, or an appropriate fraction thereof, of an active ingredient. It should be understood that in addition to the ingredients particularly mentioned above the formulations of this invention may include other agents conventional in the art having regard to the type of formulation in question, for example those suitable for oral administration may include flavouring agents. Versican V4 isoforms or inhibitors thereof can be used to provide controlled release pharmaceutical formulations containing as active ingredient one or more compounds of the invention ("controlled release formulations") in which the release of the active ingredient can be controlled and regulated to allow less frequency dosing or to improve the pharmacokinetic or toxicity profile of a given invention compound. Controlled release formulations adapted for oral administration in which discrete units comprising one or more compounds of the invention can be prepared according to conventional methods. Additional ingredients may be included in order to control the duration of action of the active ingredient in the composition. Control release compositions may thus be achieved by selecting appropriate polymer carriers such as for example polyesters, polyamino acids, polyvinyl pyrrolidone, ethylene-vinyl acetate copolymers, methylcellulose, carboxymethylcellulose, protamine sulfate and the like. The rate of drug release and duration of action may also be controlled by incorporating the active ingredient into particles, e.g. microcapsules, of a polymeric substance such as hydrogels, polylactic acid, hydroxymethylcellulose, polyniethyl methacrylate and the other above-described polymers. Such methods include colloid drug delivery systems like liposomes, microspheres, microemulsions, nanoparticles, nanocapsules and so on. Depending on the route of administration, the pharmaceutical composition may require protective coatings. Pharmaceutical forms suitable for injection include sterile aqueous solutions or dispersions and sterile powders for the extemporaneous preparation thereof. Typical carriers for this purpose therefore include biocompatible aqueous buffers, ethanol, glycerol, propylene glycol, polyethylene glycol and the like and mixtures thereof. In view of the fact that, when several active ingredients are used in combination, they do not necessarily bring out their joint therapeutic effect directly at the same time in the mammal to be treated, the corresponding composition may also be in the form of a medical kit or package containing the two ingredients in separate but adjacent repositories or compartments. In the latter context, each active ingredient may therefore be formulated in a way suitable for an administration route different from that of the other ingredient, e.g. one of them may be in the form of an oral or parenteral formulation whereas the other is in the form of an ampoule for intravenous injection or an aerosol.

### EXAMPLES

### Experimental procedures

### Tissue harvesting.

Matched cancerous and non-tumoral human breast tissue samples were obtained from mastectomy specimens. Samples were either flash-frozen in liquid nitrogen typically less than 30 minutes after the excision in the operating theatre, or directly immersed in formalin and then processed for further histological and histochemical investigations. The Ethics Committee of the University Hospital of Liege reviewed and approved the specific protocol used and written informed consent was obtained from all patients.

### PCR and sequencing.

Original primers used for PCR were previously described (Zhao and Russell, 2005). Primers used for cloning are reported in Table 1a (the position of these primers is depicted in Fig. 3a), and primers and probes used for quantitative PCR are reported in Table 1 b. PCR runs were performed with the following standard cycles: 30" 94°C, 1' 60°C, 2' 72°C, 35X (68°C for extension when using the Accuprime Pfx proofreading enzyme). Whenever required, amplicons generated with proofreading Taq were gel purified using the QiaQuick gel extraction kit (Qiagen), and cloned using the Topo-TA Cloning kit (Invitrogen). Sequencing of the purified cDNAs was performed on a 3100 Applied Biosystems sequencing unit.

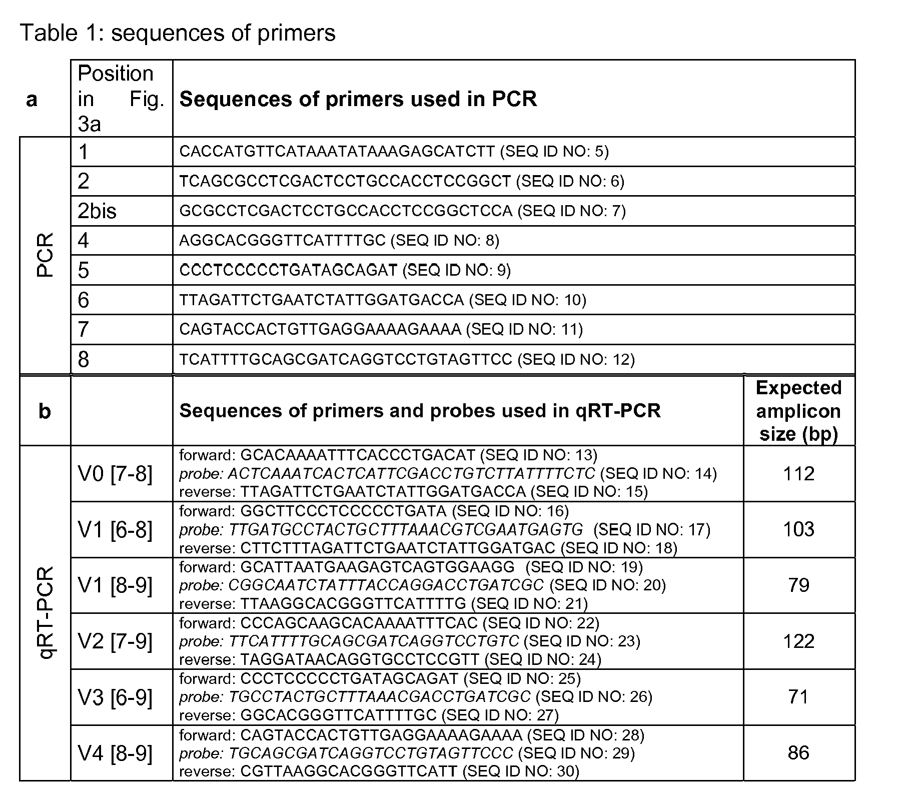

### Real Time Reverse Transcription-Polymerase Chain Reaction (RT-PCR).

Total RNA was extracted using the TriZol reagent (Invitrogen), according to the manufacturer's instructions. RNA purity was assessed both with A260/A280 ratios and quality of 18S and 28S in agarose gels. Samples with insufficient RNA quality were discarded. For cDNA synthesis, 2µg of total RNA were reverse transcribed in a 20µl reaction mixture containing 0.2µg random hexamers (GE Healthcare), 2mM of each deoxynucleotide triphosphate (dNTP, Eurogentec), 1X first strand buffer (50mM Tris/HCl pH 8.3, 75mM KCI, 3mM MgCl2, Invitrogen), 10mM dithiothreitol (Invitrogen), 40 units of recombinant ribonuclease inhibitor (Invitrogen), and 100 units of SuperScript II reverse transcriptase (Invitrogen). The RT reaction was performed at 42°C for 50' before a 15' inactivation step at 70°C. Negative controls included omission of the enzyme.

Quantitative real-time RT-PCR was performed in triplicate using the ABI Prism 7300 Sequence Detection System (Applied Biosystems), according to the manufacturer's instructions. The sequences of primers and probes for the different versican isoforms were designed using the Primer Express software (Applied Biosystems). All probes included modifications in 5' (FAM) and modifications in 3' (BHQ-1). TaqMan primers and probes for the human glyceraldehyde-3-phosphate dehydrogenase (GAPDH), β-action, 18S RNA and cyclophilin A were purchased from Applied Biosystems. All sets of primers and probes were selected to work under similar cycling conditions. cDNA samples (100ng each) were mixed with 100nM of each primer, 100nM probe and TaqMan PCR Universal Master Mix (Applied Biosystems), in a total reaction volume of 25µl. Acquired data were analysed using the Sequence Detector Software (Applied Biosystems).

### Cloning of the Versican V4 isoform.

To determine the actual molecular weight of versican V4 protein, the strategy was to generate a V4 construct. Using reverse transcription-polymerase chain reaction, total RNA from human breast tumor and the Accuprime Pfx DNA polymerase (Invitrogen), it was attempted to amplify directly the versican V4 isoform with the forward primer "1" (see Table 1 and Fig. 3a), containing the ATG start codon as well as a CACC sequence for directional cloning, and with a reverse primer including the original TGA stop codon for expression of a V4 protein without tag (primer "2") or a reverse primer allowing the addition of V5 and His tags to V4 protein (primer "2bis"). Since the forward and reverse primers are not isoform specific, and because V4 is nearly 3.2kb, V3 was preferentially amplified over V4. Therefore, V4 was generated by PCR as three smaller fragments, namely fragments A, B, and C, using reverse transcription products of RNA from breast cancer tissue. The primer pairs used were ["1" and "6"] for fragment A amplification; ["5" and "4"] for fragment B, ["7" and "2"] for fragment C. Fragments A and B were combined and amplified using primers "1" and "4" to generate fragment AB. Fragments B and C were combined and amplified using primers "5" and "2" to generate fragment BC. Finally, fragments AB and BC were combined and amplified using primers "1" and "2" (the use of primer "2bis" allowed the removal of the original TGA stop codon and ensured the presence of the carboxy-terminal V5 epitope and hexahistidine sequence) to generate the full length V4. The versican V4 coding sequence was inserted in frame into the pcDNA3.1 D/V5-His-Topo (Invitrogen). The V4 construct was checked by DNA sequencing. The same cloning procedure applied for V3, generated directly with primers "1" and "2".

### Cell Culture and expression.

NIH-3T3 cell line was originally obtained from the American Type Culture Collection (ATCC). NIH-3T3 cells were cultured in DMEM medium (Invitrogen) supplemented with 10% fetal calf serum (AEScientific) and 2mM glutamine (Invitrogen) at 37°C in a humidified 5% CO₂ incubator. NIH-3T3 cells grown in 6-wells plates were transfected at 90% confluency with 4µg of pcDNA3.1D/V5-His plasmid, containing either V4 or V3, and Lipofectamine 2000 transfection reagent (Invitrogen), according to the manufacturer's protocol. Mock transfection with transfecting reagent alone and transfection with empty pcDNA3.1D/V5-His plasmid (Invitrogen) served as controls. Cells were cultured for 48 hours, washed twice with D-PBS (Invitrogen) and lysed for total protein extraction in 1% SDS buffer containing an anti-proteases cocktail (Complete, Roche). Detection of proteins was performed either by the 12c5 antibody against the aminoterminal portion of versican (GHAP), or against the V5-tag for appropriate constructs after western blotting analysis, as detailed below. Protein concentrations were determined using BCA assay (Pierce).

### Western blotting.

Proteoglycans extraction from breast tissues was adapted from Deepa et al. (2006). Frozen tissues (from either rat brain or normal and cancer breast tissues) were pulverised using a Mikro-Dismembrator U (Braun Biotech). The obtained tissue powder was resuspended in an extraction buffer containing 50mM TBS (pH 7.0), 2mM EDTA, 10mM N-ethylmaleimide and Complete® proteases inhibitor (buffer 1), and homogenised with a tight-fitting Dounce glass homogenizer using 1ml buffer 1 per 100mg powder. The homogenate was extracted twice with buffer 1, twice with buffer 2 (buffer 1 + 0.5% Triton X-100), twice with buffer 3 (buffer 2 + 1 M NaCl) and finally with 0.5ml buffer 4 (buffer 2 + 6M urea). After extensive dialysis against chondroitinase ABC buffer (50mM Tris, 60mM sodium acetate, pH8.0), and determination of the protein content with BCA protein assay kit (Pierce), this latter extract was used for western blotting. Alternatively, it was found that direct digestion of pulverised breast tissues in chondroitinase ABC buffer worked as well. Proteins were digested at 37°C overnight with 20mU chondroitinase ABC (Sigma) per 100µg proteins. The digests were subjected to SDS-PAGE in 4-12% (NuPAGE, Invitrogen) gels under non-reducing conditions. Separated proteins were electrotransferred to nitrocellulose membranes at 160mA constant current overnight. Membranes were blocked in TBS containing 5% non-fat dried milk. Antibody 12c5 (Developmental Studies Hybridoma Bank, University of lowa) was incubated at a dilution of 1:100 2h30 at RT, followed by incubation of the membranes with a 1:8000 dilution of peroxidase-conjugated anti-mouse IgG (Santa-Cruz) for 1h. Bound antibodies were visualised using ECL chemiluminescent substrate (GE Healthcare) and exposure to X-ray films (Fuji).

### Example 1. Evidence for a new versican isoform.

Quantitative SybrGreen-based real-time RT-PCR experiments were initially performed using previously described primers (Zhao and Russell, 2005) to determine which versican isoforms are upregulated in breast cancer. The four primer pairs were first tested by classic RT-PCR, to ensure their respective specificity. Each primer pair gave the expected amplicon (∼100bp), though an unexpected band at ∼1200bp showed up with primers designed to amplify V3 (Fig. 1 b). These forward and reverse primers, located at the 3' end of exon 6 and at the 5' portion of exon 9, respectively, did not overlap exonic junctions (Fig. 1a). Theoretically, since these primers were not isoform specific, they were potentially able to generate i) a 71 bp amplicon without GAG exons corresponding to V3, as well as ii) amplicons comprising only GAGα (3032bp, V2), comprising only GAGβ (5333bp, V1) or comprising both GAG exons (8294bp, V0), provided cycling conditions allowed the polymerase to generate such longer amplicons. Since the unexpected ∼1200bp band did not correspond to an amplicon related to any known versican isoform, it was decided to perform error-free PCR amplification, cloning, and sequencing. It appeared that the primers were able to amplify not only the end of exon 6 and the beginning of exon 9, as predicted, but also the first 1194bp of exon 8 (Fig. 2). These results suggested the existence of a new versican isoform, hereafter referred to as "V4". At this stage, the most probable hypothesis was that this alternate splicing, due to a cryptic splice acceptor site inside exon 8, produced only the insertion of a segment of exon 8 between intact G1 and G3 segments, as represented in Fig. 2. Assuming the alternate splicing actually adds only 1194bp between the G1 and G3 domains, the size of V4 was expected to be 3162bp. PCR with external primers encompassing the ATG start codon (primer "1") and the TGA stop codon (primer "2", Fig. 3a) generated, in breast tumor tissues, a band at ∼2000bp (consistent with the size of V3), and only a faint, inconsistent band above 3000bp (data not shown). To ensure that V4 was not truncated, PCR was performed on different segments, in order to obtain fragments specific for the V4 isoform. The use of primers "1" and "4" generated two fragments: one strong ∼1100bp band consistent with V3 amplification (1077bp), and a fainter ∼2300bp fragment (Fig. 3b, lane 1), consistent with V4 amplification (2271 bp). The use of primer "1" and the V4 specific primer "8" (Fig. 3a) generated a ∼2300bp fragment (Fig. 3b, lane 2, the theoretical fragment size being 2261 bp). These two PCR products suggest that V4 has a classical G1 domain. Primers "5" and "2" gave three PCR products: one ∼1000bp amplicon consistent with V3 amplification, one ∼1100bp amplicon, and a ∼2200bp band also consistent with V4 having a classical G3 domain (expected size of 2160bp, Fig. 3b lane 4). Together, these results confirmed the structure of the V4 transcript depicted in Fig. 2.

Alternative splicing seems to be a process that rather frequently involves genes coding for extracellular matrix proteins.

### Example 2. mRNA analysis of versican isoforms.

In order to quantify each of these five isoforms, a series of new primers and probes was designed (Table 1b). Specific exon overlapping probes were easily found for V0, V2, V3 and V4. Based on the structure of the isoforms, no specific probe could be designed for V1. Two probes were therefore designed: a first probe overlapping exons 6 and 8 and able to amplify also V4, and a second probe overlapping exons 8 and 9 and able to amplify also V0. The quantitation of the V1 isoform was therefore made by subtracting either V4 or V0 RNA quantities from the results obtained with these two probes. From now on and for the sake of clarity, the only V1 probe that will be referred to is the probe overlapping exons 6 and 8, also able to amplify V4. Nevertheless, data consistency between the two sets of primers and probes has always been checked. Quantitative real-time RT-PCR (qRT-PCR) was performed for all versican isoforms. Results were generated from five normal-tumor paired samples for which good quality RNA was available (A260/A280 ratios > 1.8, and sharp 18S and 28S bands, as assessed by agarose gel electrophoresis). Fig. 4a shows the absolute Ct values for the 5 breast cancer samples analysed in this study and their corresponding normal breast tissues. For each isoform, Ct values were always lower in tumor samples (the lower the Ct, the higher the gene expression level). Considering all isoforms, versican Ct values were detected 10.55 ± 1.25 cycles (mean ± standard error) earlier in tumours than in their normal counterparts. It was attempted to normalise the expression levels of the different versican isoforms using various "so-called" house-keeping genes (HKG), including 18S, β-action, GAPDH, and cyclophilin A. However, despite the addition of strictly similar amounts of total RNA in the RT experiments, a high variability in the expression of HKG between matched normal and tumor samples for the same patient, as well as between samples from different patients, was noted. This variability has been previously demonstrated in breast cancer for GAPDH (Revillion et al., 2000), as well as for 9 other "reference" genes and 18S RNA (Tricarico et al., 2002). For this reason, it was decided to analyse versican isoforms expression without normalisation, keeping only "absolute" versican transcript levels. Ct values were converted in "absolute" mRNA levels (ng total RNA as deduced from a standard curve, Fig. 4b) for each isoform from the 5 normal-tumor paired samples (for V1, similar absolute values were obtained with the probe overlapping exons 8 and 9 with subtraction of absolute mRNA transcript levels obtained for V0). Fig. 4b shows that "absolute" versican transcript levels for all isoforms, including the new V4 isoform, were higher in tumor, demonstrating the overexpression of all versican isoforms in breast lesions.

### Discussion

It has been shown herein that alternate splicing can generate, at least in human breast tissues, the transcript of the new V4 isoform, which is barely detectable in normal tissues but dramatically upregulated in cancer lesions. Indeed, the present quantitative RT-PCR analyses have revealed a drastic upregulation of this new isoform. This upregulation was not specific to V4, and Fig. 4 clearly shows that all versican isoforms are upregulated in human breast cancer.

### Example 3. Western blot analyses of versican isoforms.

In this example, the expression of the various versican isoforms at the protein level in normal and adjacent tumoral breast tissues was determined. The predicted translation product of the full V4 sequence is a 118,151Da polypeptide. Cleavage of a signal sequence is predicted after alanine 20 and leucine 21 (checked also with SignaIP 3.0 webtool), and would result in a core protein of 115,737Da. The presence in V4 of a quarter of exon 8 led to speculation whether chondroitin sulfate (CS) chain addition could potentially occur at the SG or GS sequences present in the beginning of exon 8. Therefore, samples either digested or not with chondroitinase ABC were compared. The 12c5 antibody was used to reveal the versican isoforms and the degradation products bearing the aminoterminal hyaluronate-binding region of versican (HABR, see also Table 2 for a detailed list of all potential bands recognised by this antibody) expressed in normal and tumoral breast tissues.

In Table 2, the designation, predicted molecular weight and actual size in 1D-PAGE of all isoforms or G1-containing proteolytic fragments recognized by the 12c5 antibody are recited. The predicted molecular weight does not take into account the signal peptide. The actual size in gel is based on the results of the current study as well as on data available in the literature.

**Table 2:**

| Isoform or fragment | Predicted molecular weight | Actual size in gel |
|---|---|---|
| V0 | 370 | >>400 |
| V1 | 262 | >400 |
| V2 | 179 | ∼400 |
| V0 degradation product | 154 | 160 |
| V4 | 115 | ∼130 |
| V3 | 71 | ∼70 |
| V1 (or V4) degradation product | 49 | 65 |
| V2 degradation product | 42 | 64 |

Fig. 5a shows representative examples, in which major bands are numbered from 1 to 11. Chondroitinase efficiency was confirmed by the appearance of discrete high molecular weight proteins in normal and tumor samples (Fig. 5a). Adult rat brain is known to express large amounts of V2 isoform (Deepa et al., 2006). Band #3 would therefore correspond to V2 (Fig. 5b, lane 1), and bands #2 and #1 would logically correspond to V1 and V0, as per their respective increasing molecular weight. From Fig. 5a, it is therefore quite clear that V0, V1 and V2 were expressed in normal breast and overexpressed in tumor breast. It is worth noting that due to the very high molecular weight of V0, V1 and V2 species, transfer of these isoforms is most likely to be less efficient than lower molecular weight isoforms or degradation products, and caution should be taken when comparing directly the expression levels of both high and low molecular weight versican entities. Band #4 is just above 150kDa. Several studies have reported on the presence of such a band around 160kDa, which most certainly corresponds to the aminoterminal degradation product of V0 (Sandy et al., 2001). Band #9 was observed in both normal and tumoral breast tissues. Moreover, under reducing conditions, 12c5 did not recognise any versican isoform but still recognised this band #9, strongly suggesting that this band can be considered as non-specific staining. The size (in the 55kDa range) and the aspect of this latter band suggest that it represents human IgGs from breast tissues. Band #8, which migrated just above the non-specific band #9, most likely represents the Glial Hyaluronate Binding Protein (GHAP). Indeed, it has been shown that human versican V2 can be cleaved by proteases at Glu405-Gln406 to generate GHAP (Westling et al., 2004). Since only V2 is expressed in adult rat brain, the sole degradation product appearing in this lane 1 (Fig. 5b) should come from V2 and thus represent GHAP. It is noteworthy that the aminoterminal fragment arising from V1 degradation by proteases also migrates in the 65kDa range (Sandy et al., 2001). Since the lowest molecular weight versican isoform, namely V3, has a theoretical molecular weight above 70kDa, bands #10 and #11 are most probably degradation products. Similar bands were already observed with the LF99 anti-versican antibody (recognising the N terminus of all versican isoforms) in the human hippocampus, and it has been suggested that these two products presumably represent N-terminal fragments of the versican G1 domain (Westling et al., 2004). As for the three bands left (#5, #6 and #7), one is expected to correspond to V3, and another to be V4 if the protein is actually expressed. After cloning in pcDNA3.1D/V5-His and transfection, exogenous V3 and V4 were expressed in murine NIH-3T3 fibroblasts. The transcript expression of these isoforms was checked by qRT-PCR (data not shown), and whole cell lysates were analysed by western blotting (Fig. 5c). Recombinant V4 and V3 migrated closely to bands #5 and #7, respectively (lanes 1, 2, and 4, Fig. 5c). The V4 tag-expressing clone (lane 3) migrated a little bit slower than the "naked" V4, and was recognised by an anti-V5 antibody (data not shown). A smear appeared above the sharp V4 band (as well as above the tagged-V4 band), and most probably represents the processed form of V4 (with GAG added), since whole cell lysates were not digested with chondroitinase ABC. This is in good agreement with the fact that no smear appeared in the whole cell lysate from 3T3 cells overexpressing recombinant V3, since V3 does not possess any GAG attachment sites. Overall, among the 11 more frequently observed protein entities showing reactivity with the 12c5 antibody in breast cancer samples, only one of them remained difficult to identify, namely band #6, which migrated around 100kDa.

Since mRNA expression does not necessarily translate into protein expression, a further step was to unveil versican isoform expression levels with the use of the well-known 12c5 antibody after western blotting experiments. As shown in the western blots of Fig. 5a, it is obvious that the global transcript overexpression of versican isoforms in the malignant breast lesions translates into protein overexpression, since multiple bands showed up in the lesions when compared with adjacent normal breast tissues. The migration pattern of the three high molecular weight isoforms, namely V0, V1 and V2, has been described, and rat brain lysate has been used as positive control for V2. Consistent with data from the literature, these 3 isoforms migrated far above their predicted molecular weight, even after chondroitinase ABC treatment. This difference may originate either from a high content in N-and O-linked oligosaccharides, and/or from the relatively low isoelectric points of the different versican core proteins (pl< 5), which could result in reduced SDS-binding and thus may lead to aberrant gel migration properties. A tough problem when dealing with versican entities under 200kDa is to ascertain the identity of each band, since very few data are available in the literature. Identification of each band is potentially difficult, even with the use of different available antibodies, because i) as already stated above, degradation products may complicate the interpretation (12c5 recognises all degradation products containing the aminoterminal G1 domain, see Table 2), and ii) potential, not yet identified splice variants may be expressed in a tissue and/or stage-specific manner. Since V0, V1 and V2 are expressed in breast cancer, the presence of degradation products from all these isoforms may be highly suspected, and some bands recognised by 12c5 could indeed correspond to such degradation products (summarised in Table 2). This is the case for band #8, which corresponds to V2 degradation product. The V1 degradation product is expected to migrate also in the same area, and it should be noted that if proteolytic cleavage occurs in V1 at the Glu441-Ala442 bond, within the sequence DPEAAE441-A442RRGQ, the same process is likely to occur also for V4, but this assumption remains to be confirmed. V3 and V4 proteins, produced by mammalian fibroblasts transiently transfected with either human V3 and V4 cDNA-containing expression plasmids, were used to determine the actual molecular weight of these 2 isoforms, and to see whether they correspond to any of the remaining unidentified bands between 70 and 160kDa. Recombinant V3 and V4 both migrated a little bit higher than their potential human counterparts expressed in breast tumours (Fig. 5c lanes 4 and 2, respectively). This might be ascribed to the murine origin of fibroblasts, in which post-translational processing of versican proteins may differ from that occurring in human tissues. Moreover, the modification of glycosylation patterns has been largely documented in cancer development. It should also be noted that even with lysates generated from the same cells, the actual position of the V3 protein revealed with two antibodies (including 12c5) slightly differed after VEGF or TNFα stimulation in human endothelial cells. The position of the present V3 recombinant protein is otherwise in good agreement with this latter study, where V3 migration was demonstrated to be exactly between the 50 and the 75kDa molecular weight markers. Therefore, it can be safely concluded that V3 and V4 actually correspond to bands #7 and #5, respectively. Finally, no potential correspondence for band #6, located just above 100kDa, has been deduced. This band could in fact correspond to a degradation product of the high molecular weight versican entities, or to another new versican splice variant. This study presents the most comprehensive analysis to date of versican expression in human breast tissues.

### Example 4 Identification of V4 as a proteoglycan

It was determined whether V4 was a proteoglycan or simply a glycoprotein. GAG attachment is known to occur at serine-glycine residues within consensus sequences (D/E)XSG and/or SGXG. V4 features a portion of GAGβ that contains several consensus sequences, yet the answer was not obvious since the number and size of the carbohydrate chains are not only influenced by the length of the GAG binding regions, but may also be affected by the G1 (that inhibits GAG attachment) and G3 domains (that promotes GAG attachment). Moreover, versican's GAG profile is dependent on tissue type location and versican variants. It was found that the V4 band is chondroitinase ABC sensitive, yet to different degrees (see Fig. 5a). The differences seen between normal and tumor tissues range from a slightly less intense band without chondroitinase ABC treatment (lanes 8 and 9, Fig. 5a) to a black and white situation with no V4 band appearing without chondroitinase ABC treatment (lanes 2 and 3, Fig. 5a). Such differences cannot be accounted for by potential loading problems. It therefore seems that V4 is actually a proteoglycan, and this assumption is also supported by the smears only above recombinant V4 (and not V3) in NIH-3T3 cells (Fig. 5c), which indicate processing of the protein with GAG. In the present number of samples, the amount of attached GAG in human tumor samples correlates with tumor grading (data not shown).

### REFERENCES

Cattaruzza, S., Schiappacassi, M., Ljungberg-Rose, A., Spessotto, P., Perissinotto, D., Morgelin, M., Mucignat, M. T., Colombatti, A., and Perris, R. (2002) J Biol Chem 277(49), 47626-47635
Collins J., Horn N., Wadenbäck J., Szardenings M. (2001) J Biotechn 74(4):317-38
Deepa, S. S., Carulli, D., Galtrey, C., Rhodes, K., Fukuda, J., Mikami, T., Sugahara, K., and Fawcett, J. W. (2006) J Biol Chem 281 (26), 17789-17800
Dours-Zimmermann, M. T., and Zimmermann, D. R. (1994) J Biol Chem 269(52), 32992-32998
LeBaron, R. G., Zimmermann, D. R., and Ruoslahti, E. (1992), J. Biol. Chem. , 267:10003-10010
Margolis, R. U., and Margolis, R. K. (1994) Methods Enzymol 245, 105-126
Melkko, S., Scheueremann J., Dumelin CE, Neri D. (2004) Nat Biotechnol 22(5):568-574
Naso, M. F., Zimmermann, D. R., and lozzo, R. V. (1994) J Biol Chem 269(52), 32999-33008
Nimjee S.M., Rusconi C.P., Sullenger B.A. (2005) Annu Rev Med 56:555-583
Revillion, F., Pawlowski, V., Hornez, L., and Peyrat, J. P. (2000) Eur J Cancer 36(8), 1038-1042
Sandy, J. D., Westling, J., Kenagy, R. D., Iruela-Arispe, M. L., Verscharen, C., Rodriguez-Mazaneque, J. C., Zimmermann, D. R., Lemire, J. M., Fischer, J. W., Wight, T. N., and Clowes, A. W. (2001) J Biol Chem 276(16), 13372-13378
Stougaard M, Lohmann JS, Zajac M, Hamilton-Dutoit S, Koch J. BMC Biotechnol. 2007 Oct 18;7(1):69
Tricarico, C., Pinzani, P., Bianchi, S., Paglierani, M., Distante, V., Pazzagli, M., Bustin, S. A., and Orlando, C. (2002) Anal Biochem 309(2), 293-300
Westling, J., Gottschall, P. E., Thompson, V. P., Cockburn, A., Perides, G., Zimmermann, D. R., and Sandy, J. D. (2004) Biochem J 377(Pt 3), 787-795
Zako, M., Shinomura, T., Ujita, M., Ito, K., and Kimata, K. (1995) J Biol Chem 270(8), 3914-3918
Zhao, X., and Russell, P. (2005) Mol Vis 11, 603-608
Zimmermann, D. R., and Ruoslahti, E. (1989) Embo J 8(10), 2975-2981

## Claims

1. An isolated and purified nucleic acid molecule having a nucleic acid sequence encoding the V4 isoform of versican, or a fragment thereof of at least 30 nucleotides comprising the exon boundary between exons 8a and 9.

2. The nucleic acid molecule of claim 1 having the sequence of SEQ ID NO:1 or a sequence having at least 95% sequence identity therewith.

3. A method of determining the presence and/or status of a tumor in a tissue of a patient, comprising the step of determining in said tissue the presence and/or level of gene transcription of the V4 isoform of versican, whereby the presence and/or level of gene transcription of the V4 isoform of versican is indicative of the presence and/or status of said tumor.

4. The method of claim 3, which comprises comparing the level of gene transcription of the V4 isoform of versican in said tissue with the level of gene transcription in a control sample, wherein upregulation of gene transcription as compared to control is indicative of tumor formation.

5. The method of claim 4, wherein upregulation of gene transcription compared to control of at least 20% is indicative of tumor formation.

6. The method of any one of claims 3 to 5, which further comprises determining the presence and/or level of gene transcription of the V0, V1, V2 and/or V3 isoform of versican, whereby the presence and/or level of gene transcription of the V4 isoform of versican and one or more other isoforms of versican are indicative of the presence and/or status of said tumor.

7. The method of any one of claims 3 to 6, wherein the presence and/or level of gene transcription of the V4 isoform of versican is determined either at the protein level or at the gene transcription level.

8. The method of claim 7, wherein the presence and/or level of gene transcription is determined at the protein level using an anti-versican antibody or an antibody specific for the V4 isoform of versican.

9. The method of claim 8, wherein the anti-versican antibody is 12c5.

10. The method of claim 7, wherein the presence and/or level of gene transcription is determined at the gene transcription level.

11. The method of claim 10, wherein gene transcription is detected using at least one primer, probe or probe combination capable of specifically recognizing the V4 isoform of versican.

12. The method of claim 10, wherein the isoforms of versican are identified based on the size of PCR amplification products.

13. The method of claim 10, wherein gene transcription is detected using a probe or a primer combination capable of specifically hybridizing to or amplifying the exon boundary between exons 8a and 9.

14. The method of any one of claims 3 to 13, wherein the tissue is breast tissue.

15. The method of any one of claims 3 to 14, wherein the status of the tumor is further evaluated by determining the amount of glycosaminoglycan (GAG) attached to the V4 isoform of versican protein.

16. The method of any one of claims 3 to 15, wherein the tissue is selected from the group consisting of blood, tissue biopsy material or autopsy material.

17. A method for determining the presence and/or status of breast cancer in breast tissue of a patient, comprising the steps of:
(a) specifically determining in said tissue the presence and/or level of gene transcription of at least one isoform of versican selected from the group consisting of the V0 isoform, the V1 isoform, the V2 isoform, the V3 isoform, and the V4 isoform;
(b) determining the presence and/or status of said tumor based on the result from step (a).

18. The method of claim 17, wherein the presence and/or level of gene transcription is determined at the protein level.

19. A method of screening for an agent which modulates the expression of the V4 isoform of versican, comprising:
(a) contacting host cells which comprise a nucleic acid molecule encoding the V4 isoform of versican with candidate molecules; and
(b) determining whether the candidate molecules modulate the expression of the V4 isoform.

20. An oligonucleotide capable of specific recognition of the V4 isoform of versican by specifically hybridizing to the exon boundary between exons 8a and 9.

21. A kit for detecting the presence and/or status of a tumor in a tissue, comprising at least one of the following:
- A probe or primer pair capable of specifically detecting the V4 isoform of versican by specifically hybridizing to or amplifying the exon boundary between exons 8a and 9; or
- an antibody specific for the V4 isoform of versican.

22. A vector comprising the nucleic acid molecule of claim 1 or 2.

23. A method for producing the V4 isoform of versican or a fragment thereof of at least 100 nucleotides comprising the exon boundary between exons 8a and 9, comprising:
- providing a host cell;
- transforming the host cell with an isolated and purified nucleic acid molecule having a nucleic acid sequence encoding the V4 isoform of versican or a fragment thereof of at least 100 nucleotides comprising the exon boundary between exons 8a and 9;
- culturing the host cell so that the host cell expresses the V4 isoform of versican or the fragment thereof of at least 100 nucleotides comprising the exon boundary between exons 8a and 9.

24. An isolated V4 isoform protein or peptide comprising a sequence of at least 10 amino acids which is encoded by a nucleotide sequence comprising the exon boundary between exons 8a and 9.

25. The V4 isoform protein or peptide comprising a sequence having at least 70% sequence identity with SEQ ID NO:2.

26. The V4 isoform of versican or a fragment thereof for use as a medicament.

27. The V4 isoform of versican or a fragment thereof for use in the treatment of breast cancer.

28. Use of the V4 isoform of versican or a fragment thereof for the manufacture of a medicament for the treatment of breast cancer.

29. An inhibitor of the V4 isoform of versican for use as a medicament, wherein the inhibitor of the V4 isoform of versican is selected from an inhibitory antibody specific for the V4 isoform of versican, a small molecule inhibitor of the V4 isoform of versican or an antisense nucleic acid molecule directed against the V4 isoform of versican.

30. An inhibitor of the V4 isoform of versican for use in the treatment of breast cancer, wherein the inhibitor of the V4 isoform of versican is selected from an inhibitory antibody specific for the V4 isoform of versican, a small molecule inhibitor of the V4 isoform of versican or an antisense nucleic acid molecule directed against the V4 isoform of versican.

31. Use of an inhibitor of the V4 isoform of versican for the manufacture of a medicament for the treatment of breast cancer, wherein the inhibitor of the V4 isoform of versican is selected from an inhibitory antibody specific for the V4 isoform of versican, a small molecule inhibitor of the V4 isoform of versican or an antisense nucleic acid molecule directed against the V4 isoform of versican.

32. A ligand binding specifically to the V4 isoform of versican, wherein said ligand is coupled to a bioactive agent or diagnostic agent.

33. A method for targeting an anti-cancer drug to a breast tumor in a patient, said method comprising the steps of linking the anti-cancer drug to a ligand specific for the V4 isoform of versican and administering the ligand so obtained to the patient.
